(19) Europäisches Patentamt
European Patent Office
Office européen des brevets

(11) EP 1 280 843 B1

(12) **FASCICULE DE BREVET EUROPEEN**

(45) Date de publication et mention de la délivrance du brevet:
**18.08.2010 Bulletin 2010/33**

(21) Numéro de dépôt: **01928038.7**

(22) Date de dépôt: **23.04.2001**

(51) Int Cl.:
***C08G 61/12*** *(2006.01)* ***H01B 1/12*** *(2006.01)*

(86) Numéro de dépôt international:
**PCT/FR2001/001241**

(87) Numéro de publication internationale:
**WO 2001/081446 (01.11.2001 Gazette 2001/44)**

(54) **COMPLEXE ELECTROACTIF, SONDE ELECTROACTIVE ET PROCEDE DE PREPARATION**

ELEKTROAKTIVER KOMPLEX, ELEKTROAKTIVE SONDE UND VERFAHREN ZUR HERSTELLUNG

ELECTROACTIVE COMPLEX, ELECTROACTIVE PROBE AND PREPARATION METHOD

(84) Etats contractants désignés:
**AT BE CH CY DE DK ES FI FR GB GR IE IT LI LU MC NL PT SE TR**

(30) Priorité: **21.04.2000 FR 0005195**

(43) Date de publication de la demande:
**05.02.2003 Bulletin 2003/06**

(73) Titulaire: **BIO MERIEUX**
**69280 Marcy l'Etoile (FR)**

(72) Inventeur: **GARNIER, Francis**
**F-94500 CHAMPIGNY SUR MARNE (FR)**

(74) Mandataire: **Guerre, Dominique et al**
**Cabinet Germain et Maureau**
**12 Rue Boileau**
**B.P. 6153**
**69466 Lyon Cedex 06 (FR)**

(56) Documents cités:
**WO-A-00/31750 WO-A-90/10655**
**WO-A-95/29199**

- **GARNIER F ET AL: "TOWARD INTELLIGENT POLYMERS: DNA SENSORS BASED ON OLIGONUCLEOTIDE -FUNCTIONALIZED POLYPYRROLES" SYNTHETIC METALS,CH, LAUSANNE, vol. 100, no. 1, 1999, pages 89-94, XP000889886 ISSN: 0379-6779**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 280 843 B1

## Description

L'invention a trait aux électrodes organiques élaborées à partir de polymères électroactifs sur lesquels sont liés des anti-ligands destinés à interagir spécifiquement avec des ligands.

L'interaction spécifique de l'anti-ligand avec le ligand entraîne une variation sensible et sélective des propriétés électrochimiques du polymère électroactif, telle qu'une diminution de l'électroactivité dudit polymère. Cette variation, qui dépend de la concentration en ligand greffé, est observée, éventuellement mesurée, et directement corrélée à la quantité de ligand greffé. Une des applications essentielles de cette technique réside donc dans la détection, l'identification, et éventuellement le dosage, d'un ligand, présent dans un échantillon biologique.

La variation précitée est de type potentiométrique, telle qu'une variation du potentiel d'oxydation du polymère électroactif avant et après interaction, ou de type ampérométrique, telle qu'une variation du courant d'oxydation ou de réduction du polymère avant et après hybridation déterminé à un potentiel déterminé.

Pour caractériser précisément la réponse électrochimique du polymère, celui-ci doit présenter une forte électroactivité.

C'est donc un des buts de l'invention d'apporter une sonde à base de polymère électroactif sur lequel est fixé au moins un anti-ligand, cette sonde possédant une forte électroactivité.

Selon le document WO-A-95/29199, on connaît un polypyrrole, constitué par des monomères consistant chacun en un noyau pyrrole substitué de manière covalente sur le carbone en position 3 du noyau pyrrole, par un polynucléotide sonde.

Selon le document WO 00/31750 A1, on connaît des complexes électroactifs pour lesquels le polymère électroactif est un polypyrrole lié de manière covalente, par l'intermédiaire d'un groupe $(CH2)_m$ CONH $(CH2)_{m''}$ où m et m" sont identiques ou différents et sont un nombre entier compris entre 1 et 3, à un groupement électrodonneur consistant en un ferrocène, une quinone, un ferrocène ou une quinone activé(e) par un ester sélectionné dans le groupe consistant en COON-hydroxyphtalamide, COON-hydroxysuccinimide et COOpentafluorophénol, ledit groupement électrodonneur étant lui-même lié de manière covalente à un anti-ligand qui interagit spécifiquement avec un ligand.

Le polypyrrole ainsi obtenu est appliqué à la détection, et éventuellement le dosage, de ligands, in vitro ou in vivo.

On est cependant toujours dans l'attente de l'élaboration de polymères possédant de meilleures propriétés électroactives.

Selon l'invention, on apporte un polymère électroactif, modifié, porteur d'au moins un anti-ligand, et dont la sensibilité peut être de l'ordre

de cent fois supérieure à celle d'un polymère électroactif modifié connu, tel qu'un polypyrrole qui fait l'objet du document WO-A-95/29199.

Ainsi, un premier objet de l'invention est un complexe électroactif, constitué par un polymère homopolymère ou copolymère électroactif d'au moins deux monomères, un anti-ligand et un ligand ayant spécifiquement interagi avec ledit anti-ligand, ledit complexe comprenant en outre au moins un groupe électrodonneur, ledit complexe électroactif étant tel que revendiqué à la revendication 1.

Selon l'invention, le groupement électrodonneur est avantageusement choisi parmi le ferrocène, la quinone et les dérivés de ceux-ci, et/ou le polymère électroactif est de préférence choisi parmi le polypyrrole, le polyacétylène, la polyazine, le poly(p-phénylène), le poly(p-phénylène vinylène), le polypyrène, le polythiophène, le polyfuranne, le polysélénophène, la polypyridazine, le polycarbazole, la polyaniline, les polynucléotides bicaténaires.

Avant de détailler l'invention, certains termes employés dans la description et les revendications sont ci-après définis.

Par « groupement électrodonneur », on entend un couple redox présentant une vague d'oxydation étroite, rapide et réversible, tel que le ferrocène, la quinone et leurs dérivés, par exemple leurs dérivés substitués.

Les termes « anti-ligand » et « ligand » font indifféremment référence à des molécules biologiques telles que des polynucléotides ou des peptides, mais aussi à des molécules chimiques.

L'anti-ligand est susceptible d'interagir spécifiquement avec le ligand pour former un conjugué ligand / anti-ligand. A titre d'exemples de conjugués, on peut citer tout couple peptide / anticorps, anticorps / haptène, hormone / récepteur, polynucléotide / polynucléotide, polynucléotide / acide nucléique et analogues.

Le terme « polynucléotide » tel qu'employé selon l'invention désigne un enchaînement d'au moins cinq nucléotides (désoxyribonucléotides ou ribonucléotides), naturels ou modifiés, susceptible de s'hybrider, dans des conditions appropriées d'hybridation, avec un polynucléotide au moins partiellement complémentaire. Par nucléotides modifiés, on entend par exemple, un nucléotide comportant une base modifiée et/ou comportant une modification au niveau de la liaison internucléotidique et/ou au niveau du squelette. A titre d'exemple de bases modifiées, on peut citer l'inosine, la méthyl-5-désoxycytidine, la diméthylamino-5-désoxyuridine, la diamino-2,6-purine et la bromo-5-désoxyuridine. Pour illustrer une liaison internucléotidique modifiée, on peut mentionner les liaisons phosphorothioate, H-phosphonate et alkyl-phosphonate. Les alpha-oligonucléotides tels que ceux décrits dans FR-A-2 607 507 et les PNA qui font l'objet de l'article de M. Egholm et al., J. Am. Chem. Soc. (1992) 114, 1895-1897, sont des exemples de polynucléotides constitués

de nucléotides dont le squelette est modifié.

Le terme « peptide » signifie notamment tout enchaînement d'au moins deux acides aminés, tels que protéine, fragment de protéine, oligopeptide, qui a été extrait, séparé, isolé ou synthétisé, comme un peptide obtenu par synthèse chimique ou par expression dans un organisme recombinant. Sont inclus aussi, tout peptide dans la séquence duquel un ou plusieurs acides aminés de la série L sont remplacés par un ou plusieurs acides aminés de la série D, et vice-versa ; tout peptide dont l'une au moins des liaisons CO-NH est remplacée par une liaison NH-CO ; tout peptide dont l'une au moins des liaisons CO-NH est remplacée par une liaison NH-CO, la chiralité de chaque résidu aminoacyle, qu'il soit impliqué ou non dans une ou plusieurs dites liaisons CO-NH, étant soit conservée, soit inversée par rapport aux résidus aminoacyles constituant un peptide de référence (ou immunorétroïdes) ; et tout mimotope.

Pour illustrer les diverses classes des peptides concernés, on peut mentionner les hormones adrénocorticotropiques ou leurs fragments, les analogues d'angiotensine et leurs inhibiteurs, les peptides natriurétiques, la bradykinine et ses dérivés peptidiques, les peptides chimiotactiques, la dynorphine et ses dérivés, les endorphines et leurs dérivés, les encéphalines et leurs dérivés, les inhibiteurs d'enzymes, les fragments de fibronectine et leurs dérivés, les peptides gastro-intestinaux, les peptide opioïdes, l'oxytocine, la vasopressine, la vasotocine et leurs dérivés, les protéines kinase.

Le terme « anticorps » définit tout anticorps monoclonal ou polyclonal, tout fragment d'undit anticorps tel que les fragments Fab, Fab'2 ou Fc, ainsi que tout anticorps obtenu par modification ou recombinaison génétique.

Les termes « greffer », « lier » et « fixer » en l'absence de toute indication, sont indifféremment employés dans le présent texte pour désigner une relation entre deux entités, sans en définir la nature chimique. Il peut ainsi s'agir d'une liaison faible ou d'une liaison covalente.

Un groupe de liaison selon l'invention relie, par liaison covalente, deux entités chimiques, après interaction desdites deux entités, l'une au moins ayant été au préalable activée ou activable, en vue de cette interaction, par un groupe activé ou activable. Le groupe de liaison peut donc résulter de la réaction d'undit groupe activé ou activable d'une entité sur une fonction réactive de l'autre entité, et vice-versa, ou de la réaction d'undit groupe activé ou activable d'une entité sur un autre dit groupe activé ou activable de l'autre entité.

Par groupe activé, on entend un groupe permettant, par son intermédaire, l'interaction de l'entité sur lequel il est fixé avec une autre entité. A titre d'exemple, il peut s'agir d'un groupe ester activé tel que le groupe -CO-[O-N-phtalimide]. Par groupe activable, on comprend un groupe qui peut être transformé en un groupe activé, par exemple dans certaines conditions réactionnelles ou lors de la mise en contact avec un groupe activé susceptible d'interagir avec lui.

Le complexe de l'invention tel que défini précédemment répond avantageusement aux caractéristiques suivantes considérées seules ou en combinaison.

Le ligand et l'anti-ligand sont des molécules biologiques, notamment choisies parmi les polynucléotides et les polypeptides, qui peuvent être marqués par un traceur susceptible de générer directement ou indirectement un signal.

Le complexe présente l'une des structures suivantes : le groupement électrodonneur est lié, directement ou indirectement, d'une part au polymère électroactif et d'autre part à l'anti-ligand ou au ligand, ou bien le groupement électrodonneur est lié, directement ou indirectement, à l'anti-ligand, ledit anti-ligand étant lui-même lié, directement ou indirectement, au polymère électroactif, ou bien le groupement électrodonneur est lié, directement ou indirectement, au ligand ayant interagi avec l'anti-ligand, ledit anti-ligand étant lié, directement ou indirectement, au polymère électroactif.

Lorsque le groupement électrodonneur est lié au polymère électroactif, il l'est préférentiellement de manière covalente, par l'intermédiaire d'un premier groupe de liaison.

Lorsque l'anti-ligand ou le ligand sont liés au groupement électrodonneur et/ou au polymère électroactif, ils le sont avantageusement de manière covalente, par l'intermédiaire respectivement d'un second et d'un troisième groupes de liaison.

Les premier et/ou second et/ou troisème groupes de liaison relient respectivement, le groupe électrodonneur au polymère électroactif, le groupe électrodonneur à l'anti-ligand ou au ligand, et l'anti-ligand ou le ligand au polymère électroactif, par l'intermédiaire d'un bras de couplage.

Dans la structure du complexe de l'invention, selon laquelle le groupe électrodonneur est lié au ligand, il peut aussi l'être par l'intermédiaire d'un support inerte, par exemple une bille de polystyrène, une bille magnétique ou une bille de verre, ou d'un support biologique, telle qu'une cellule, dans laquelle le groupe électrodonneur a été internalisé.

Selon un complexe préféré de l'invention, le polymère électroactif est un polypyrrole constitué d'au moins deux monomères consistant chacun en un noyau pyrrole, et le groupe électrodonneur est le ferrocène, et, en particulier, l'anti-ligand est un polynucléotide sonde et le ligand est un polynucléotide cible, au moins partiellement hybridé audit anti-ligand.

Ce complexe préféré présente en outre les caractéristiques suivantes, considérées seules ou en combinaison.

Le ferrocène est lié d'une part au polynucléotide sonde et d'autre part au noyau pyrrole d'un monomère du polypyrrole, auquel cas le polynucléotide sonde est fixé sur le carbone en position 1 d'un des noyaux cyclopentadiène du ferrocène, et le ferrocène est fixé sur le noyau pyrrole par l'intermédiaire du carbone en position 1' de l'autre noyau du cyclopentadiène,
ou bien

le polynucléotide sonde est lié au ferrocène et au noyau pyrrole dudit monomère au moins, auquel cas le polynucléotide sonde est fixé sur le carbone en position 1 d'un des noyaux cyclopentadiène du ferrocène.

Le noyau pyrrole est de préférence substitué sur le carbone en position 3.

Le polypyrrole est un copolymère et comprend un monomère dont le noyau pyrrole est substitué par un groupe $-CH_2-COOH$ ou $-CH_2-CH_2OH$.

Avantageusement, le premier groupe de liaison entre le ferrocène et le noyau pyrrole, est le groupe $-CONH-CH_2-$ et/ou le second groupe de liaison, entre le ferrocène et le polynucléotide sonde ou le polynucléotide cible est le groupe -CO- et/ou le troisième groupe de liaison entre le polynucléotide sonde ou le polynucléotide cible et le noyau pyrrole, est le groupe $-CH_2-CO-$.

Les premier et/ou second et/ou troisième groupes de liaison qui relient, indirectement, respectivement le ferrocène au noyau pyrrole, le ferrocène au polynucléotide sonde ou au polynucléotide cible et le noyau pyrrole au polynucléotide sonde ou au polynucléotide cible, peuvent le faire par l'intermédiaire d'un bras de couplage. Celui-ci est avantageusement une chaîne hydrocarbonée saturée ayant au moins deux atomes de carbone, de préférence au moins 2 ou 3 atomes de carbone.

Le polynucléotide sonde ou le polynucléotide cible est fixé au ferrocène et/ou au noyau pyrrole du monomère au moins, par l'intermédiaire respectivement du second et/ou du troisième groupes de liaison et d'au moins une des fonctions aminées du polynucléotide sonde ou du polynucléotide cible.

Un autre objet de l'invention est une sonde électroactive, telle que revendiquée à la revendication 23, constituée par un polymère homopolymère ou copolymère, électroactif, d'au moins deux monomères, un anti-ligand susceptible d'interagir spécifiquement avec un ligand, ladite sonde comprenant en outre au moins un groupement électrodonneur.

Elle possède avantageusement au moins l'une quelconque des caractéristiques suivantes.

Le polymère électroactif est choisi parmi le polypyrrole, le polyacétylène, la polyazine, le poly(p-phénylène), le poly(p-phénylène vinylène), le polypyrène, le polythiophène, le polyfuranne, le polysélénophène, la polypyridazine, le polycarbazole, la polyaniline, les polynucléotides bicaténaires et/ou le groupe électrodonneur est choisi parmi le ferrocène, la quinone et les dérivés de ceux-ci, et/ou l'anti-ligand est une molécule biologique notamment choisie parmi les polynucléotides et les polypeptides, et est éventuellement marqué par un traceur susceptible de générer directement ou indirectement un signal.

Le groupement électrodonneur est lié, directement ou indirectement, d'une part au polymère électroactif et d'autre part à l'anti-ligand. Dans ce cas, le groupement électrodonneur est de préférence lié de manière covalente au polymère électroactif, par l'intermédiaire d'un premier groupe de liaison, ou bien

le groupement électrodonneur est lié, directement ou indirectement, à l'anti-ligand, ledit anti-ligand étant lui-même lié, directement ou indirectement, au polymère électroactif.

L'anti-ligand est lié de manière covalente au groupement électrodonneur, par l'intermédiaire d'un second groupe de liaison et/ou l'anti-ligand est lié de manière covalente au polymère électroactif, par l'intermédiaire d'un troisième groupe de liaison.

Les premier et/ou second et/ou troisème groupes de liaison relient respectivement, le groupe électrodonneur au polymère électroactif, le groupe électrodonneur à l'anti-ligand et l'anti-ligand au polymère électroactif, par l'intermédiaire d'un bras de couplage.

Une sonde avantageuse de l'invention comprend un polypyrrole constitué d'au moins deux monomères consistant chacun en un noyau pyrrole, le ferrocène, et un anti-ligand particulier consistant en un polynucléotide sonde, susceptible d'hybrider un polynucléotide cible, dans des conditions d'hybridation appropriées.

Le polynucléotide sonde ($PN_{sonde}$) peut être fixé entre le ferrocène (Fe) et le noyau pyrrole (P) dudit monomère au moins, ou bien être fixé sur le ferrocène, celui-ci étant fixé sur le noyau pyrrole.

Lorsque le polynucléotide sonde est fixé entre le ferrocène et le noyau pyrrole, selon une structure que l'on désignera par $P-PN_{sonde}-Fe$, la sonde de l'invention répond aux caractéristiques suivantes considérées seules ou combinées :

le polynucléotide sonde est fixé sur le carbone en position 1 d'un des noyaux cyclopentadiène du ferrocène ;
le polynucléotide sonde est, directement ou indirectement, fixé au noyau pyrrole, par l'intermédiaire d'un troisième groupe de liaison ; celui-ci est avantageusement le groupe $-CH_2-CO-$.

Lorsque le polynucléotide sonde est fixé sur le ferrocène lui-même étant fixé sur le noyau pyrrole, selon une structure que l'on désignera par $P-Fe-PN_{sonde}$, la sonde de l'invention répond aux caractéristiques suivantes considérées seules ou combinées :

le polynucléotide sonde est fixé sur le carbone en position 1 d'un des noyaux cyclopentadiène du ferrocène, et le ferrocène est fixé sur le noyau pyrrole par l'intermédiaire du carbone en position 1' de l'autre noyau du cyclopentadiène ;

le ferrocène est, directement ou indirectement, fixé au noyau pyrrole dudit monomère au moins, par l'intermédiaire d'un premier groupe de liaison ; celui-ci est avantageusement le groupe -CONH-$CH_2$-.

Quelle que soit la structure de la sonde, le ferrocène est, directement ou indirectement, fixé au polynucléotide sonde, par l'intermédiaire d'un second groupe de liaison qui consiste de préférence en le groupe -CO-.

Par ailleurs, le polynucléotide sonde est fixé au noyau pyrrole du monomère au moins et/ou au ferrocène, par l'intermédiaire respectivement du troisième et/ou du second groupes de liaison et l'intermédiaire d'au moins une des fonctions aminées du polynucléotide sonde.

Selon une structure avantageuse d'une sonde de l'invention, les premier et/ou second et/ou troisième groupes de liaison précités, relient, indirectement, respectivement le ferrocène au noyau pyrrole, le polynucléotide sonde au noyau pyrrole et le polynucléotide sonde au noyau pyrrole, par l'intermédiaire d'un bras de couplage.

Ce bras de couplage est de préférence une chaîne hydrocarbonée saturée ayant au moins deux atomes de carbone, de préférence au moins 2 ou 3 atomes de carbone.

Le polypyrrole modifié selon l'invention peut être un homopolymère ou un copolymère. Le noyau pyrrole est substitué sur le carbone en position 3, donc tout copolymère de l'invention présentera au moins deux monomères dont un au moins n'est pas substitué sur ladite position, ou bien il présentera au moins deux monomères différemment substitués sur ladite position, les substituants $PN_{sonde}$ étant différents et/ou l'assemblage des substituants $PN_{sonde}$ et Fe étant différents sur le noyau pyrrole des monomères et/ou les premier et/ou second et/ou troisième groupes de liaison et/ou les bras de couplage étant différents. En plus des monomères modifiés selon l'invention, un copolymère peut comprendre au moins un monomère dont le noyau pyrrole est substitué par un groupe - $CH_2$-COOH ou -$CH_2$-$CH_2$OH.

Lorsque tous les monomères du polymère sont modifiés à l'identique, on obtient un polypyrrole homopolymère de l'invention.

L'invention concerne aussi un procédé pour préparer une sonde de l'invention.

Si les monomères modifiés du polypyrrole ont tous la structure

P-$PN_{sonde}$-Fe, le procédé comprend alors les étapes suivantes :

(a) On dispose d'un polypyrrole homopolymère ou copolymère constitué par au moins deux monomères consistant chacun en un noyau pyrrole, dont au moins un est substitué sur le carbone en position 3 par un polynucléotide sonde,
(b) On obtient le ferrocène comportant sur le carbone en position 1 d'un des noyaux cyclopentadiène, au moins un groupe activé ou activable, et
(c) On met en contact le polypyrrole homopolymère ou copolymère, substitué par un polynucléotide sonde, avec le ferrocène comportant ledit groupe activé ou activable.

Si les monomères modifiés du polypyrrole ont tous la structure P-Fe-$PN_{sonde}$, le procédé comprend alors les étapes suivantes :

(a) On dispose de ferrocène comportant au moins deux groupements activés ou activables, au moins l'un étant fixé sur le carbone en position 1 d'un des noyaux cyclopentadiène et l'autre étant fixé sur le carbone en position 1' de l'autre noyau cyclopentadiène du ferrocène,
(b) On dispose d'un monomère consistant en un noyau pyrrole substitué sur le carbone en position 3, par un groupe activé ou activable,
(c) On fait réagir un excès du ferrocène comportant des groupes activés ou activables avec le monomère substitué,
(d) On effectue une électropolymérisation du composé obtenu en (c), et
(e) On met en contact le polymère obtenu à l'étape (d) avec un polynucléotide sonde.

Les procédés de préparation de sonde de l'invention sont en outre avantageusement caractérisés comme suit :

Le ou les groupes activés ou activables du ferrocène, identiques ou différents, sont de préférence un groupe ester activé ou activable, et de préférence le groupe -CO-[N-hydroxy-phtalimide] ; ils sont avantageusement fixés sur le ferrocène par l'intermédiaire d'un bras de couplage.

Le groupe activé du noyau pyrrole est -$CH_2$-$NH_2$.

Le groupe activé ou activable du noyau pyrrole, est fixé sur celui-ci par l'intermédiaire d'un bras de couplage.

Un bras de couplage préféré est une chaîne hydrocarbonée saturée ayant au moins deux atomes de carbone, de préférence au moins 2 ou 3 atomes de carbone.

L'étape d'électropolymérisation est réalisée en utilisant des techniques bien connues de l'homme du métier. Par exemple, elle peut être conduite en soumettant les monomères à des variations de potentiel électrique suffisantes pour provoquer la polymérisation par une oxydation et une réduction successives ; ou bien par polymérisation à courant (chronopotentiométrie) ou à potentiel (chronoampérométrie) imposés.

Un autre objet de l'invention est un composé ligand / ferrrocène, en tant que produit intermédiaire, consistant

en un ligand lié, directement ou indirectement, à un groupe électrodonneur tel que le ferrocène, la quinone ou les dérivés de ceux-ci. En particulier, -ce composé est un composé polynucléotide / ferrocène, en tant qu'intermédiaire de préparation d'une sonde de l'invention dans le procédé de préparation d'une sonde P-$PN_{sonde}$-Fe de l'invention. Il consiste en un polynucléotide fixé sur le carbone en position 1 d'un des noyaux cyclopentadiène du ferrocène, par l'intermédiaire d'un second groupe de liaison qui est de préférence le groupe -CO-.

Ce second groupe de liaison peut relier indirectement le ferrocène et le polynucléotide sonde, par l'intermédiaire d'un bras de couplage qui consiste avantageusement en une chaîne hydrocarbonée saturée ayant au moins deux atomes de carbone, de préférence au moins 2 ou 3 atomes de carbone.

Une sonde de l'invention présente des applications diagnostiques. Aussi, l'invention concerne encore un procédé de détection d'un polynucléotide cible dans un échantillon biologique, selon lequel on met en contact ladite sonde, dans des conditions d'hybridation appropriées, et on met en évidence ou on quantifie une différence de potentiel ou une variation de courant entre la sonde avant mise en contact et la sonde après mise en contact. Ledit procédé de détection étant tel que revendiqué à la revendication 45.

L'invention concerne également l'utilisation pour la détection d'un polynucléotide cible dans un échantillon biologique d'une sonde, utilisation selon laquelle on met en contact ladite sonde, dans des conditions d'hybridation appropriées, et on met en évidence ou on quantifie une différence de potentiel ou une variation de courant entre la sonde avant mise en contact et la sonde après mise en contact.

La présente invention a aussi pour objet une électrode dont tout ou partie de la surface est revêtue d'une sonde définie précédemment. Une telle électrode peut être obtenue par toute technique classique bien connue de l'homme du métier. A titre d'exemple, il peut être procédé à cette préparation en déposant un polypyrrole de l'invention à la surface d'une électrode de platine, d'or, de chrome ou de titane recouvert d'or, de carbone vitreux ou d'un oxyde conducteur tel que l'oxyde d'étain ou un oxyde mixte d'étain et d'indium.

L'invention concerne aussi l'utilisation d'un groupe électrodonneur pour augmenter l'électroactivité d'un polymère électroactif sur lequel est fixé un anti-ligand susceptible d'interagir avec un ligand, ledit groupe électrodonneur se trouvant sur le même monomère que l'anti-ligand.

Les différents objets de l'invention sont illustrés dans les exemples suivants qui font référence aux figures 1 à 15 annexées et desquels ressortiront leurs caractéristiques préférentielles et avantages.

### Exemple 1 : Préparation d'un polypyrrole copolymère constitué de monomères de structure P-$PN_{sande}$-Fe et de monomères substitués par le groupe -$CH_2$-COOH

L'élaboration de cette structure a nécessité la synthèse d'un ferrocène substitué par un groupe N-hydroxyphtalimide (en abrégé NHP), et la fixation du polynucléotide sonde sur un polypyrrole, puis la fixation du ferrocène activé obtenu sur le polynucléotide sonde fixé sur le polypyrrole.

### 1 A-Obtention du ferrocène activé, le 1-ferrocène-propyl-NHP

Le schéma de synthèse est le suivant :

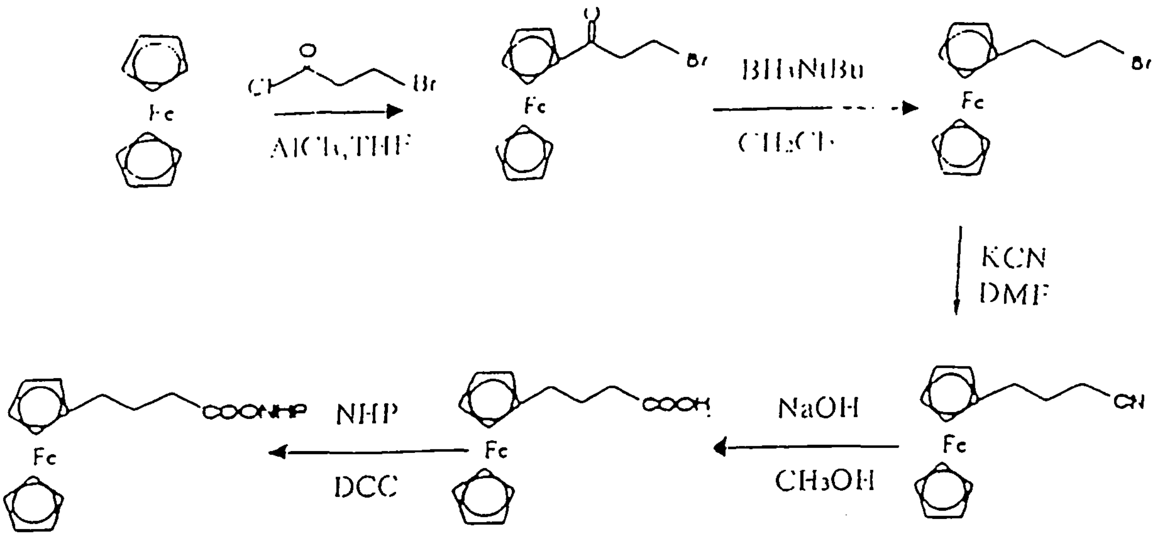


L'analyse de la réponse électrochimique du 1-ferrocène-propyl-NHP ainsi obtenu met en évidence un système électrochimique réversible situé à un potentiel de 0,3 V/ECS. Cette réponse comparée à celle d'un ferrocène substitué directement par un -groupe NHP montre un gain en potentiel de l'ordre de 200mV.

**1B-Fixation du polynucléotide sonde sur le polypyrrole copolymère [PyCOOH,PyCONH-NHP]**

Cette étape est réalisée dans les mêmes conditions que celles décrites dans le document WO-A-95/29199, en utilisant un polynucléotide sonde fonctionnalisé sur ses extrémités 3' et 5' par des groupes aminés, l'un des groupes servant à la fixation sur le polymère polypyrrole activé et l'autre à la fixation sur le ferrocène activé.

**1C-Fixation du ferrocène sur le polypyrrole copolymère poly[PyCOOH,Py-$PN_{sonde}$]**

Le greffage du $PN_{sonde}$ a été effectué en plongeant une électrode de poly[PyCOOH,Py-$PN_{sonde}$] dans une solution contenant du 1-ferrocène-propyl-NHP obtenu en 1A, pendant 1 heure à température ambiante. L'électrode est ensuite rincée et analysée électrochimiquement dans une solution de 0,5M NaCl.

La réponse représentée à la Figure 1 (en trait plein, Fe-$(CH_3)$NHP, en trait pointillé, Fe-NHP) est caractérisée par un signal électrochimique apparu après greffage du $PN_{sonde}$ à un potentiel de 0,35 V/ECS en milieu aqueux caractéristique du ferrocène substitué sur le $PN_{sonde}$. La mesure de la charge échangée au cours de l'oxydation montre la présence de sites de ferrocène électroactif. Cette mesure permet de déterminer la quantité totale de ferrocène fixé sur le $PN_{sonde}$.

**Exemple 2 : Utilisation du polypyrrole obtenu à l'exemple 1 pour hybrider un polynucléotide cible ($PN_{cible}$)**

L'hybridation est effectuée en plaçant l'électrode recouverte du polypyrrole obtenu à l'exemple 1, pendant 1 heure dans un tampon PEG en présence de 25 nmoles de $PN_{cible}$ à 37°C. L'électrode est ensuite rincée et analysée par voltamétrie cyclique.

La réponse électrochimique représentée par la figure 2 (en trait plein, avant hybridation, en trait pointillé, après hybridation) montre une forte diminution de l'électroactivité du signal du ferrocène après hybridation. Le courant diminue de 70 $\mu$A. Cette forte diminution démontre la forte sensibilité du ferrocène à l'hybridation de $PN_{cible}$.

Pour optimiser ce polypyrrole, il est nécessaire de définir un seul site de greffage sur le $PN_{sonde}$ pour contrôler la position et la quantité du ferrocène greffé, pour conduire à une parfaite reproductibilité des résultats. Cette optimisation fait l'objet de l'exemple 3.

**Exemple 3 : Préparation d'un polypyrrole copolymère constitué de monomères de structure P-Fe-$PN_{sonde}$**

Cette préparation a consisté en la fonctionnalisation du polypyrrole par un ferrocène portant un groupe partant. Un $PN_{sonde}$ a été fixé d'une part sur l'homopolymère [PyNH-Fe-NHP] et d'autre part sur le copolymère [PyNH-Fe-NHP-PyCOOH].

Le $PN_{sonde}$ présente des propriétés de reconnaissance vis-à-vis d'un $PN_{cible}$ et on a vérifié si cette reconnaissance était conservée après fixation du $PN_{sonde}$ sur le ferrocène, puis on a recherché la nature de la réponse électrochimique du ferrocène à cette reconnaissance.

On a d'abord effectué la synthèse d'un monomère pyrrole substitué en position 3 par un ferrocène portant un groupe ester activé. Puis, on a réalisé une polymérisation par voie électrochimique du monomère obtenu, et on a greffé le $PN_{sonde}$ sur le polymère résultant.

**3A-Synthèse du monomère, le 3-pyrrole-ferrocène-NHP**

L'élaboration d'un pyrrole qui porte à la fois un ferrocène et un groupe NHP a nécessité plusieurs étapes de synthèse. La première est la synthèse de pyrrole substitué par un groupe butyle amine, soit le (4-aminobutyl)-3-pyrrole, la seconde est la synthèse de 1,1'-dipropanoate-N-hydroxyphtalamide-ferrocène, et la troisième étape est la synthèse du 3-pyrrole-ferrocène-NHP.

Synthèse du (4-aminobutyl)-3-pyrrole

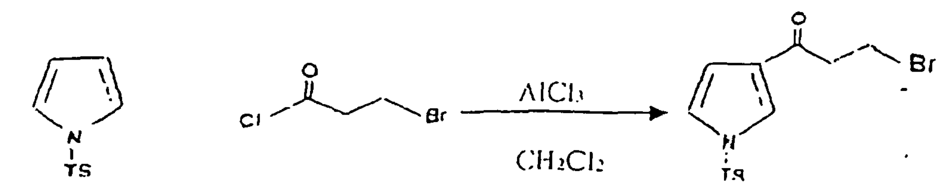


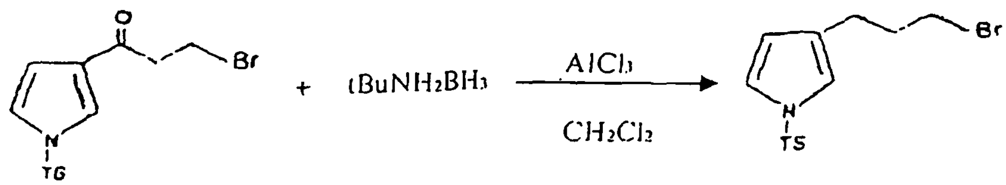


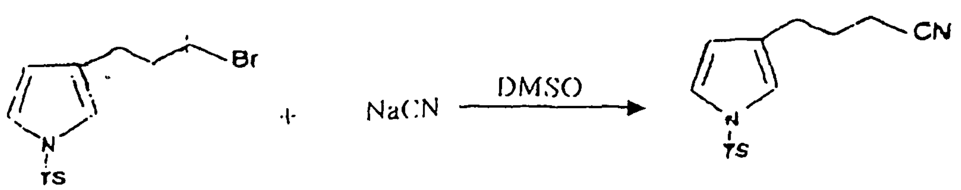

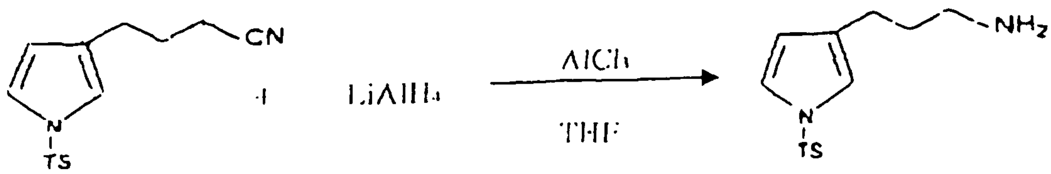


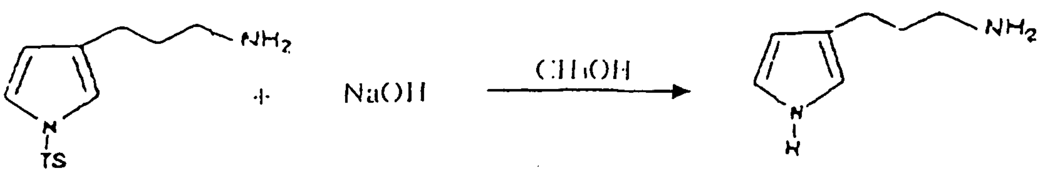


Le (4-amino butyle)-3-pyrrole dont la fonction amine permet la fonctionnalisation du pyrrole par des groupes ester activé grâce aux réactions de condensation est synthétisé. Pour synthétiser ce monomère, on effectue une réaction d'acylation sur le pyrrole tosyle pour obtenir le (3-bromo-propionyl)-3-tosyle-1-pyrrole avec un rendement de 63%. Cette réaction est suivie d'une réduction de la fonction carbonyle. On obtient le 1-tosyle-pyrrole-3-bromopropyle avec un rendement de 78%. En utilisant le cyanure de sodium, le brome est substitué par une fonction cyanure avec un rendement de 50%. La transformation du cyanure en amine se fait par réaction de réduction en utilisant l'hydrure d'aluminium et de lithium (rendement 77%). L'hydrolyse du groupement tosyle par une base NaOH conduit au (4-amino butyle)-3-pyrrole avec un rendement de 62%.

Synthèse du 1,1'ferrocène propyl NHP

Le schéma de synthèse est le suivant :

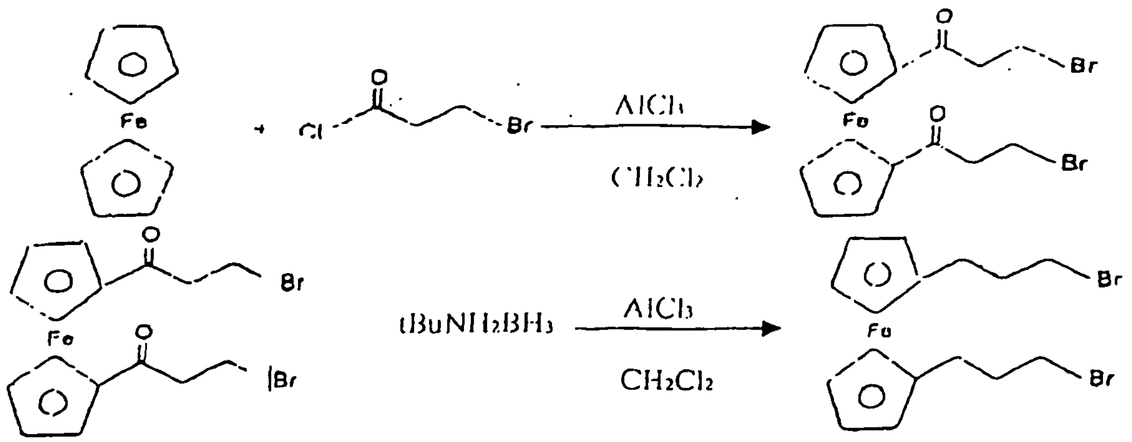

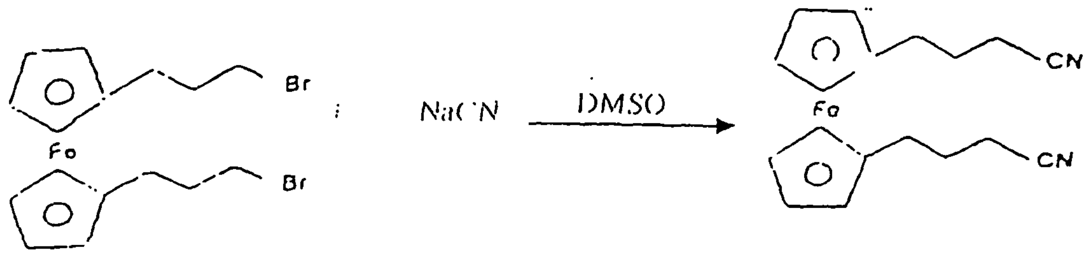


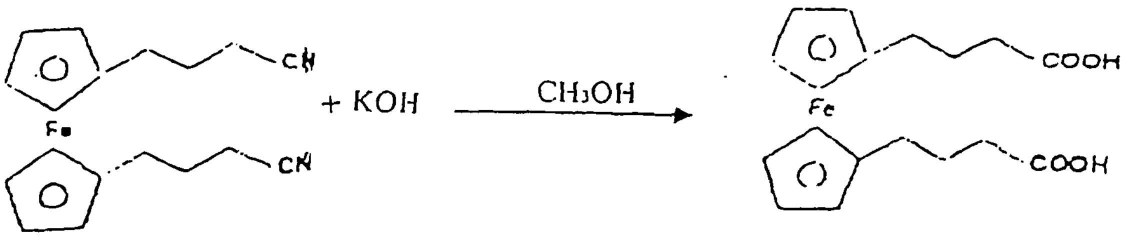


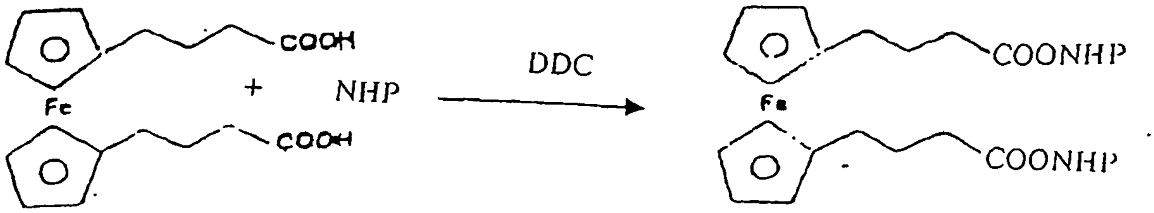


Ce ferrocène est obtenu par la transformation de 1,1'-ferrocène-propyl-cyano en 1,1'-ferrocène-propyl-acide en présence d'hydroxyde de sodium et de méthanol avec un rendement de 95%. Les groupements carboxyliques du ferrocène sont substitués par un groupe partant, le N-hydroxyphtalimide (NHP). Le ferrocène NHP est obtenu à partir du ferrocène acide, par estérification et en présence de DCC. Le DCC, joue un rôle de déshydratant, se transforme en DCU en captant ainsi l'eau formée au cours de la réaction.

Synthèse du 3-pyrrole-ferrocène-NHP

Le schéma de synthèse est le suivant :

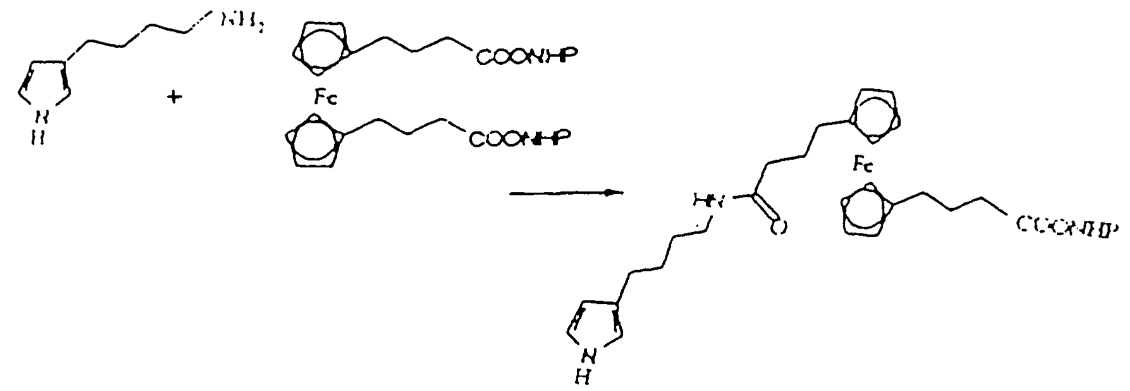

Le 3-pyrrole-ferrocène-NHP est obtenu par une réaction de couplage entre le 3-pyrrole-butylamine et le 1,1'ferrocène-propyl-NHP dans l'acétonitrile, dans des conditions de large excès en ferrocène pour éviter une substitution du pyrrole sur les deux groupes NHP du ferrocène.

Caractérisation électrochimique du monomère

L'analyse électrochimique du monomère en solution est effectuée dans l'acétonitrile en présence d'électrolyte support 0,1 M $LiClO_4$.

La figure 3 montre le voltamogramme obtenu. Deux systèmes d'oxydoréduction sont observés. Le premier système présente des caractéristiques réversibles dont le potentiel d'oxydation est situé à 0,26 V. Ce système correspond à l'oxydoréduction du ferrocène substitué sur le pyrrole. Le deuxième système électrochimique est irréversible, son potentiel d'oxydation est situé à 1,17 V/ECS, il correspond à l'oxydation du pyrrole.

Ce potentiel d'oxydation du pyrrole substitué par le groupe ferrocène est élevé comparé au potentiel d'oxydation de pyrrole non substitué ou substitué avec le groupe carboxylique qui est généralement de l'ordre de 0,8 à 0,9 V/ECS.

**3B-Préparation de l'homopolymère poly[Py-Fe-NHP]**

Le schéma de polymérisation est le suivant :

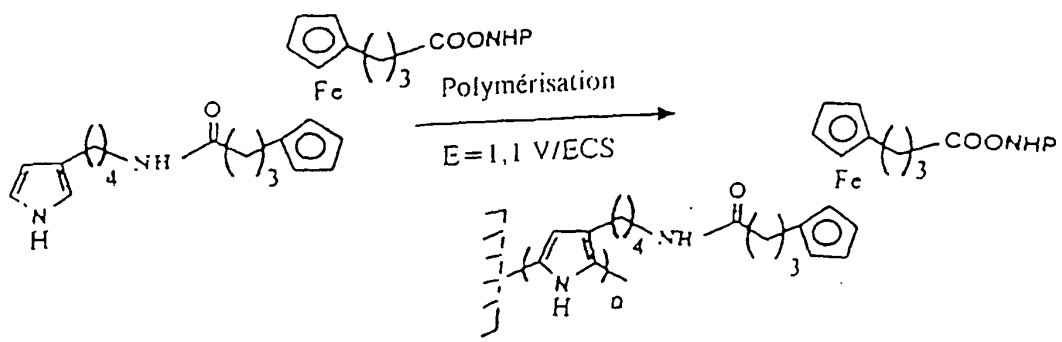


Les films de poly[Py-Fe-NHP] sont obtenus en plongeant les électrodes de platine dans une solution du monomère poly[Py-Fe-NHP] en milieu acétonitrile qui contient 0,2M de tétrafluoroborate de tétrabutylammonium, ($N^+Bu_2BF_4$). L'électropolymérisation est effectuée à un potentiel contrôlé de 1,1 V/ECS en se basant sur le potentiel d'oxydation du monomère. La charge déposée est de 70 MC.

*Après polymérisation, il se forme un précité dans la solution qui est probablement dû à la formation des oligomères de courtes chaînes.*

Caractérisation électrochimique de l'homopolymère poly[Py-Fe-NHP]

Les films obtenus d'homopolymère poly[Py-Fe-NHP,PyCOOH] sont analysés électrochimiquement dans un milieu acétonitrile en présence de 0,1M de $LiClO_4$. La figure 4, représente le voltamogramme obtenu. Ce voltamogramme montre une grande électroactivité du film de polymère. Le potentiel d'oxydation est de 288 mV/ECS et celui de la réduction est de 236 mV/ECS. La différence entre ces deux valeurs et le rapport des courants des pics d'oxydation (13,5 µA) et de réduction (11 µA) montrent que ce système électrochimique présente une bonne réversibilité. La charge échangée au cours de l'oxydation ou de la réduction est de 0,1 MC. Cette valeur est très faible comparée à la charge déposée au cours de la polymérisation. A partir de la charge d'oxydation, on peut déterminer la quantité de ferrocène électroactive déposée sur l'électrode. Sachant que le ferrocène échange un électron au cours de son processus d'oxydation, on peut établir la loi de faraday :

$$Q_{ox} = N(Fe)*F$$

Le nombre de moles de ferrocène ou d'unités pyrrole électroactives sur l'électrode est de $10^{-9}$ moles.
Le calcul du nombre de mole de ferrocène à partir de la charge de polymérisation montre des valeurs très élevées de l'ordre de $10^{-7}$ moles.
*Cependant, ces résultats montrent que le rendement de polymérisation est très faible. Deux facteurs sont à l'origine de ce faible rendement, d'une part, une partie de la charge de polymérisation sert à l'oxydation du ferrocène en solution et d'autre part, l'encombrement stérique du groupement ferrocène-NHP substitué sur le pyrrole empêche le couplage entre ces deux unités pyrrole et conduit à des oligomères de courtes chaînes qui précipitent en solution.*

**3C-Fixation du $PN_{sonde}$ sur l'homopolymère**

Le schéma de fixation est le suivant :

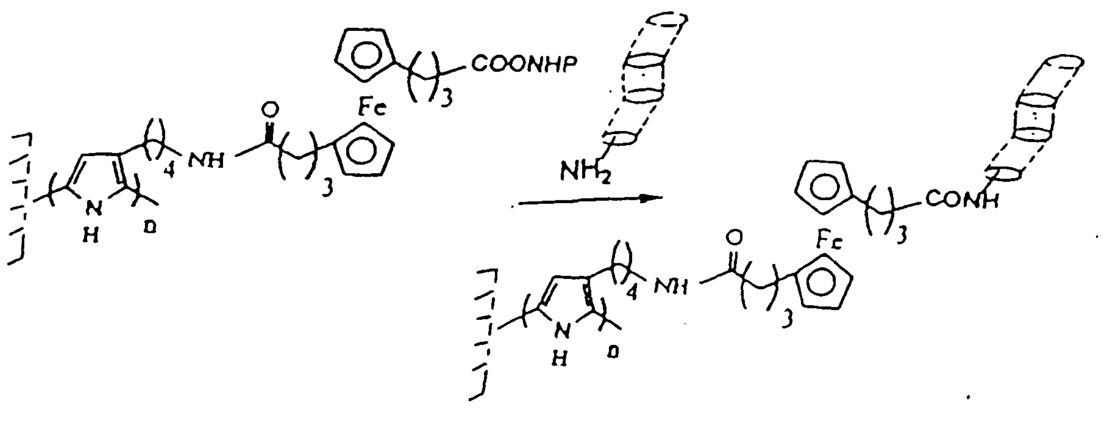

Le $PN_{sonde}$ utilisé est un polynucléotide de 25 paires de bases ayant la séquence suivante identifiée par SEQ ID NO :1 :

$^{5'}$TCA-ATC-TCG-GGA-ATC-TCA-ATG-TTA-G$^{3'}$.

La fixation du $PN_{sonde}$ est effectué sur l'homopolymère précurseur poly[Py-Fe-NHP]. Les conditions chimiques de cette substitution sont similaires à celles de la fixation sur le polymère poly[PyCCNH-NHP]
Le couplage entre le pyrrole Fe-NHP et le $PN_{sonde}$ portant le groupe amine dans sa position 5' est effectué en plongeant l'électrode dans une solution d'acétonitrile en présence de 10% d'un tampon acétate et de 25 µmole/l. La réaction se déroule à température ambiante pendant 2 heures.
Les films obtenus sont analysés par voltamétrie cyclique dans l'eau en présence de 0,5M NaCl. La figure 5 montre un système d'oxydoréduction dont les pics d'oxydation et de réduction sont situés respectivement à 273 mV et

258 mV.

Le système électrochimique du ferrocène en milieu aqueux n'est pas aussi réversible comparé à l'analyse du film dans l'acétonitrile. Cette perte de l'électroactivité est probablement liée à des problèmes de solvatation soit de l'ion dopant ou de la matrice de polmymère dans l'eau.

**Exemple 4 : Hybridation du $PN_{cible}$ sur l'homopolymère poly[Py-Fe-$PN_{sonde}$] obtenu à l'exemple 3**

Le schéma d'hybridation est le suivant :

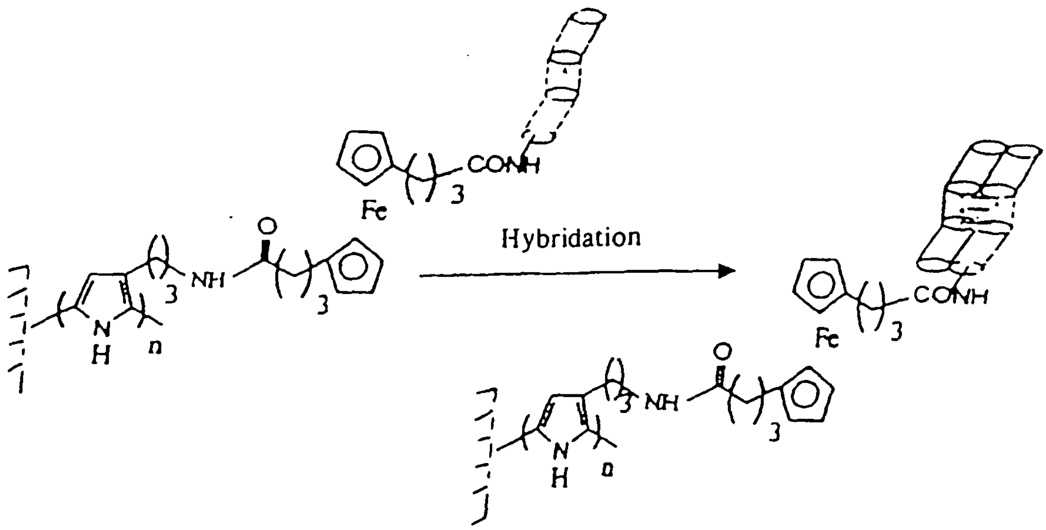


Des films de copolymère poly[Py-Fe-$PN_{sonde}$] ont été mis en incubation en présence de 25 nmoles (25 µmol/l) de $PN_{cible}$ dans un tampon biologique, pendant 3 heures, à une température de 37°C, dans un volume de 1 ml.

Le $PN_{cible}$ comporte 25 bases complémentaires des bases du $PN_{sonde}$ et répond à la séquence suivante, identifiée par SEQ ID NO :2

$^{5'}$CTA-ACA-TTG-AGA-TTC-CCG-AGA-TTG-A$^{3'}$.

Un test de blanc est effectué en incubant une autre électrode dans le tampon d'hybridation qui contient de l'ADN de saumon pour déterminer les effets des interactions non spécifiques.

Caractérisation électrochimique

La réponse électrochimique des polymères a été analysée par voltamétrie cyclique en milieux aqueux en présence de 0,5M NaCl.

De l'électrode en blanc

Le voltamogramme apparaissant à la figure 6 (en trait plein, avant hybridation, en trait pointillé, après hybridation) montre la réponse électrochimique de l'électrode de référence incubée en présence de tampon PEG.

On observe que l'électrode qui sert de référence montre une variation du potentiel d'oxydation et une diminution de l'électroactivité. Ceci indique que les interactions non spécifiques ont un léger effet sur la réponse électrochimique du ferrocène.

Du Polymère testé après hybridation

L'analyse électrochimique de l'électrode incubée en présence du $PN_{cible}$ en milieux aqueux est représentée sur la figure 7 (en trait plein : avant hybridation, en trait pointillé : après hybridation).

L'électrode après incubation montre un déplacement du potentiel d'oxydation vers les potentiels élevés et une diminution de l'électroactivité. La charge échangée au cours de l'oxydation est de la réduction diminue également.

On observe que la variation de la réponse électrochimique du ferrocène est plus importante comparée à l'électrode de blanc.

**Exemple 5 : Préparation d'un polypyrrole copolymère constitué de monomères de structure P-Fe-NHP et de monomères substitués par le groupe -$CH_2$-COOH**

**5A-Préparation du polymère poly[Py-Fe-NHP,PyCOOH]**

Le schéma de synthèse est le suivant :

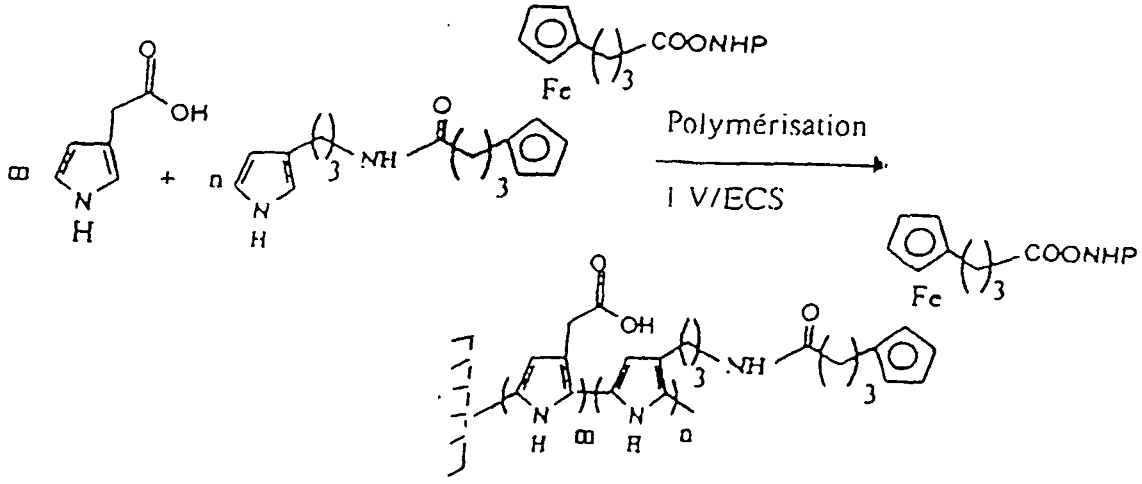


La copolymérisation entre le 3-pyrrole-acide acétique (PyCOOH) et le 3-pyrrole-Fe-NHP (Py-Fe-NHP) permet de diluer ce monomère encombrant dans la matrice de polymère afin de minimiser les perturbations d'origines stériques. En plus la copolymérisation avec le 3-pyrrole-acide acétique acide permet d'augmenter la porosité des films.

Le choix de concentrations respectives de monomères PyCOOH et Py-Fe-NHP est déterminé en se référant aux potentiels de polymérisation de ces deux monomères. Le PyCOOH s'oxyde à 0,9 V/ECS, le Py-Fe-NHP s'oxyde à 1,1 V/ECS. Il est nécessaire de prendre une faible concentration de PyCOOH par rapport au Py-Fe-NHP. Les concentrations de ces deux monomères utilisées pour 1'électropolymérisation du copolymère sont donc 0,02 M pour le PyCOOH et 0,08 M pour le Py-Fe-NHP.

Le copolymère a été déposé à potentiel imposé de 1,1 V/ECS sur une électrode de platine de surface de 700 $cm^2$ en milieu propylène carbonate en présence de 0,2 M de $LiCl_2$. La charge déposée au cours de l'électropolymérisation est de 35 MC. Il est difficile de déterminer l'épaisseur du film à partir de la charge de polymérisation. Puisqu'une partie de la charge mesurée au cours de l'électropolymérisation a servi à l'oxydation du ferrocène en solution.

Caractérisation électrochimique du polymère poly[Py-Fe-NHP,PyCOOH] obtenu

Cette caractérisation a été effectuée en milieu acétonitrile en présence de 0,1 M $LiClO_4$. Le voltamogramme en figure 8, montre un système électrochimique réversible avec un pic d'oxydation situé à un potentiel de 254 mV et un pic de réduction symétrique situé à un potentiel de 234 mV. La largeur à mi-hauteur est de 150 mV. Cette réponse électrochimique présente le signal du ferrocène et montre une électroactivité importante de cette sonde électrochimique. Le transfert électronique à travers la chaîne conjuguée du polymère est très important, ce qui montre que le polypyrrole est conducteur. La charge échangée au cours de l'oxydation du ferrocène est de l'ordre de 0,15 mC. Cette charge d'oxydation du ferrocène permet de calculer le nombre de moles de pyrroles portant le groupe ferrocène dans le polymère en supposant que tous les sites ferrocène sont électroactifs.

A partir de la loi de Faraday on a : .

$Q_{ox}$ = N*F avec N le nombre de mole de ferrocène et F la constante de Faraday.

Le nombre de mole de ferrocène électroactive est de l'ordre de $10^{-9}$ mole.

**5B-Fixation du polynucléotide sonde sur le polypyrrole copolymère poly[Py-Fe-NHP,PyCOOH]**

Le schéma de fixation est le suivant :

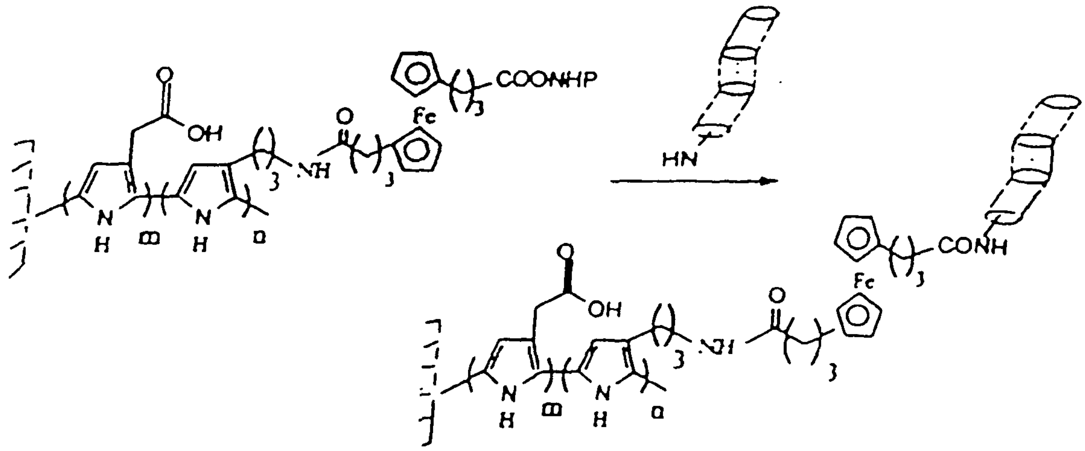

Le $PN_{sonde}$ utilisé est le polynucléotide SEQ ID NO :1.

La fixation du $PN_{sonde}$ (25µmoles/l) est effectuée sur le copolymère précurseur poly[Py-Fe-NHP,PyCOOH] en plongeant l'électrode dans une solution d'acétonitrile en présence de 10% de tampon acétate.

Caractérisation électrochimique du polymère poly[Py-Fe-$PN_{sonde}$,PyCOOH] obtenu

La figure 9 montre la réponse électrochimique de l'électrode après greffage du $PN_{sonde}$ en milieux aqueux en présence de 0,5 M NaCl. Le voltamogramme présente un système d'oxydoréduction caractérisé par un pic d'oxydation intense et fin et un pic de réduction large.

Les potentiels des pics d'oxydation et de réduction sont respectivement de 341 mV et 275 mV, la largeur à mi-hauteur est de 100 mV pour le pic d'oxydation et de 200 mV pour le pic de réduction. La charge échangée au cours de l'oxydation ou de la réduction est de l'ordre de 0,1 mC.

**Exemple 6 : Hybridation du PNcible sur le copolymère poly[Py-Fe-$PN_{sonde}$,PyCOOH] obtenu à l'exemple 5**

Le schéma d'hybridation est le suivant :

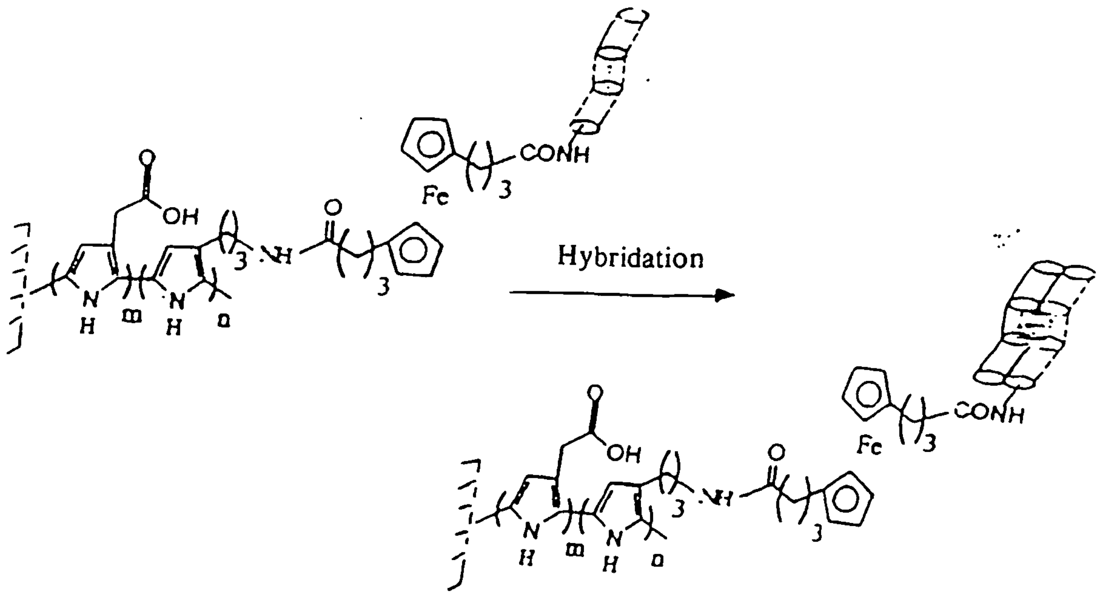


Des films de copolymère poly[Py-Fe-$PN_{sonde}$'PyCOOH] ont été mis en incubation en présence de $PN_{cible}$ de 25 bases dans les mêmes conditions que précédemment.

Une électrode de référence est incubée dans le même tampon biologique sans $PN_{cible}$.

Caractérisation électrochimique

La réponse électrochimique des électrodes incubées en présence de la cible et de la non-cible a été analysée en milieu acétonitrile et en milieux aqueux.

De l'électrode en blanc

Les figures 10 et 11 représentent les voltamogrammes avant (en trait plein) et après (en trait pointillé) hybridation en présence de la non-cible (ADN de saumon) en milieu acétonitrile et en milieu aqueux.

On observe qu'en milieu acétonitrile, la réponse électrochimique du ferrocène montre une stabilité du potentiel d'oxydation avec une très légère variation des courants de pics.

L'analyse de la réponse électrochimique de l'électrode de référence en milieu aqueux en présence de 0,5 M NaCl montre une variation de la réponse électrochimique du ferrocène. Une augmentation du potentiel d'oxydation et une diminution du courant des pics.

Du copolymère testé après hybridation

L'analyse électrochimique de l'électrode incubée en présence de la cible en milieux aqueux est représentée sur la figure 12 (en trait plein : avant hybridation, en trait pointillé : après hybridation). Le voltamogramme montre une forte variation du signal électrochimique après hybridation. Le potentiel d'oxydation se déplace vers les potentiels les plus élevés ; il passe de 340 mV à 387 mV après hybridation. Le courant d'oxydation diminue de 60%. La charge échangée au cours de l'oxydation diminue de 25%.

L'analyse de la réponse électrochimique dans l'acétonitrile de l'électrode incubée en présence de 0,1 nmole de $PN_{cible}$ est représentée sur la figure 13 (en trait plein : avant hybridation, en trait pointillé : après hybridation). Le voltamogramme après incubation montre la sauvegarde du système réversible du ferrocène en milieu organique. Le potentiel d'oxydation et de réduction du ferrocène se déplace vers les bas potentiels.

Cette variation de la réponse électrochimique en milieu aqueux et en milieu organique permet de montrer qu'il existe une hybridation sélective et spécifique entre le $PN_{sonde}$ greffé sur le ferrocène et le $PN_{cible}$.

Bien que ces électrodes incubées en présence de la cible aient montré des variations significatives de la réponse électrochimique, il est nécessaire de déterminer l'origine de la variation de la réponse du ferrocène après incubation dans un tampon biologique sans la cible.

### Exemple 6 : Influence de la charge de polymérisation sur l'électroactivité

L'influence de la charge déposée au cours de la polymérisation sur la variation du signal électrochimique du poly[py-Fe-$PN_{sonde}$,PyCOOH] de l'exemple 5 a été étudiée pour trois valeurs de charges 5, 10 et 15 mC.
La figure 14 démontre que le courant d'oxydation diminue avec la quantité de la charge déposée.

### Exemple 7 : Reproductibilité électrochimique des électrodes

La reproductibilité électrochimique du poly[Py-Fe-$PN_{sonde}$,PyCOOH] de l'exemple 5 a été testée sur six électrodes de films de faible épaisseur.
La caractérisation électrochimique de ces électrodes réalisée en milieu acétonitrile en présence de 0,1 M $LiClO_4$, est illustrée à la figure 15 qui montre qu'on obtient sensiblement les mêmes voltamogrammes, après greffage du PNsonde pour les six électrodes.

### Exemple 8 : Influence des conditions d'hybridation du $PN_{cible}$ sur le $PN_{sonde}$

#### 8A-Effet du tampon d'hybridation

La même électrode poly[Py-Fe-$PN_{sonde}$,PyCOOH] (Q = 5 mC) de l'exemple 5 a été incubée dans deux tampons différents :

- Tampon TE-NaCl à 37°C pendant 2 heures et 3 heures,
- Tampon SSPEG à 37°C pendant 2 heures et 3 heures.

La caractérisation électrochimique (voltamogrammes non représentés) effectuée en milieu acétonitrile en présence de 0,1 M $LiClO_4$ respectivement, avant et après l'incubation pour chacun des tampons ci-dessus met en évidence l'intérêt du tampon SSPEG dans lequel l'électrode est plus stable.

#### 8B-Effet de la concentration du $PN_{cible}$

Plusieurs films de poly[Py-Fe-$PN_{sonde}$,PyCOOH] de l'exemple 5 ont été incubés dans un tampon SSPEG, en présence de différentes concentrations de $PN_{cible}$ (0,01, 0,05 et 0,5 nmoles/l)
La caractérisation électrochimique (voltamogrammes non représentés) effectuée en milieu acétonitrile en présence de 0,1 M $LiClO_4$ respectivement, avant et après hybridation en présence des différentes concentrations ci-dessus, montre que la variation de la réponse électrochimique avant et après hybridation, augmente avec la concentration du $PN_{cible}$. On observe en effet qu'au potentiel de 280 mV/ECS qui correspond au potentiel du pic initial, la diminution du courant varie avec la concentration du $PN_{cible}$.

## Revendications

1. Complexe électroactif, constitué par un polymère homopolymère ou copolymère électroactif d'au moins deux monomères, un anti-ligand et un ligand ayant spécifiquement interagi avec ledit anti-ligand, **caractérisé en ce qu'**il comprend en outre au moins un groupe électrodonneur et **en ce qu'**il présente l'une des trois structures suivantes :

   - le groupement électrodonneur est lié de manière covalente d'une part au polymère électroactif et d'autre part à l'anti-ligand qui interagit spécifiquement avec le ligand ou au ligand qui interagit spécifiquement avec l'anti-ligand,
   - le groupement électrodonneur est lié de manière covalente à l'anti-ligand qui interagit spécifiquement avec le ligand, ledit anti-ligand étant lui-même lié de manière covalente au polymère électroactif,
   - le groupement électrodonneur est lié de manière covalente au ligand qui interagit spécifiquement avec l'anti-ligand, ledit anti-ligand étant lié de manière covalente au polymère électroactif,

   étant exclus les complexes électroactifs pour lesquels ledit polymère électroactif est un polypyrrole lié de manière covalente, par l'intermédiaire d'un groupe $(CH2)_m$ CONH $(CH_2)_{m''}$ où m et m" sont identiques ou différents et sont un nombre entier compris entre 1 et 3, à un groupement électrodonneur consistant en un ferrocène, une quinone, un ferrocène ou une quinone activé(e) par un ester sélectionné dans le groupe consistant en COON-hydroxyphtalamide, COON-hydroxysuccinimide et COOpentafluorophénol, ledit groupement électrodonneur étant lui-même lié

de manière covalente à un anti-ligand qui interagit spécifiquement avec un ligand.

2. Complexe selon la revendication 1, **caractérisé en ce que** le polymère électroactif est choisi parmi le polypyrrole, le polyacétylène, la polyazine, le poly(p-phénylène), le poly(p-phénylène vinylène), le polypyrène, le polythiophène, le polyfuranne, le polysélénophène, la polypyridazine, le polycarbazole, la polyaniline, les polynucléotides bicaténaires.

3. Complexe selon la revendication 1, **caractérisé en ce que** le groupe électrodonneur est choisi parmi le ferrocène, la quinone et les dérivés de ceux-ci.

4. Complexe selon la revendication 1, **caractérisé en ce que** le ligand et l'anti-ligand sont des molécules biologiques notamment choisies parmi les polynucléotides et les polypeptides.

5. Complexe selon la revendication 4, **caractérisé en ce que** le ligand et/ou l'anti-ligand sont marqués par un traceur susceptible de générer directement ou indirectement un signal.

6. Complexe selon la revendication 1, **caractérisé en ce que** l'anti-ligand ou le ligand sont liés de manière covalente au groupement électrodonneur, par l'intermédiaire d'un second groupe de liaison.

7. Complexe selon la revendication 1, **caractérisé en ce que** l'anti-ligand ou le ligand sont liés de manière covalente au polymère électroactif, par l'intermédiaire d'un troisième groupe de liaison.

8. Complexe selon l'une quelconque des revendications 6 à 7, **caractérisé en ce que** les second et/ou troisième groupes de liaison relient respectivement, le groupe électrodonneur à l'anti-ligand et l'anti-ligand au polymère électroactif, par l'intermédiaire d'un bras de couplage.

9. Complexe selon la revendication 1, **caractérisé en ce que** le groupe électrodonneur est lié au ligand, par l'intermédiaire d'un support inerte ou biologique.

10. Complexe selon la revendication 9 **caractérisé en ce que** le support inerte est une bille de polystyrène, une bille magnétique ou une bille de verre.

11. Complexe selon la revendication 9, **caractérisé en ce que** le support biologique est une cellule, dans laquelle le groupe électrodonneur a été internalisé.

12. Complexe selon la revendication 1, **caractérisé en ce que** le polymère électroactif est un polypyrrole constitué d'au moins deux monomères consistant chacun en un noyau pyrrole, et **en ce que** le groupe électrodonneur est le ferrocène.

13. Complexe selon la revendication 12, **caractérisé en ce que** l'anti-ligand est un polynucléotide sonde et le ligand est un polynucléotide cible, au moins partiellement hybridé audit anti-ligand.

14. Complexe selon la revendication 13, **caractérisé en ce que** le polynucléotide sonde est lié au ferrocène et au noyau pyrrole dudit monomère au moins.

15. Complexe selon la revendication 12, **caractérisé en ce que** le noyau pyrrole est substitué sur le carbone en position 3.

16. Complexe selon la revendication 14, **caractérisé en ce que** le polynucléotide sonde est fixé sur le carbone en position 1 d'un des noyaux cyclopentadiène du ferrocène.

17. Complexe selon la revendication 12, **caractérisé en ce que** le polypyrrole est un copolymère et comprend un monomère dont le noyau pyrrole est substitué par un groupe $-CH_2-COOH$ ou $-CH_2-CH_2OH$.

18. Complexe selon la revendication 14, **caractérisé en ce que** le second groupe de liaison, entre le ferrocène et le polynucléotide sonde ou le polynucléotide cible est le groupe -CO-.

19. Complexe selon la revendication 14, **caractérisé en ce que** le troisième groupe de liaison entre le polynucléotide sonde ou le polynucléotide cible et le noyau pyrrole, est le groupe $-CH_2-CO-$.

**20.** Complexe selon l'une quelconque des revendications 18 à 19, **caractérisé en ce que** les second et/ou troisième groupes de liaison relient, indirectement, le ferrocène au polynucléotide sonde ou au polynucléotide cible et le noyau pyrrole au polynucléotide sonde ou au polynucléotide cible, par l'intermédiaire d'un bras de couplage.

**21.** Complexe selon la revendication 20, **caractérisé en ce que** le bras de couplage est une chaîne hydrocarbonée saturée ayant au moins deux atomes de carbone, de préférence au moins 2 ou 3 atomes de carbone.

**22.** Complexe selon l'une quelconque des revendications 13 à 21, **caractérisé en ce que** le polynucléotide sonde ou le polynucléotide cible est fixé au ferrocène et/ou au noyau pyrrole du monomère au moins, par l'intermédiaire respectivement du second et/ou du troisième groupes de liaison et d'au moins une des foncions aminées du polynucléotide sonde ou du polynucléotide cible.

**23.** Sonde électroactive pour la détection et/ou l'identification d'un ligand, constituée par un polymère homopolymère ou copolymère électroactif d'au moins deux monomères et un anti-ligand, **caractérisée en ce qu'**elle comprend en outre au moins un groupe électrodonneur et **en ce qu'**elle présente l'une des deux structures suivantes :

- le groupement électrodonneur est lié de manière covalente d'une part au polymère électroactif et d'autre part à l'anti-ligand,
- le groupement électrodonneur est lié de manière covalente à l'anti-ligand, ledit anti-ligand étant lui-même lié de manière covalente au polymère électroactif,

étant exclues les sondes électroactives pour lesquelles ledit polymère électroactif est un polypyrrole lié de manière covalente, par l'intermédiaire d'un groupe $(CH2)_m$ CONH $(CH2)_{m'}$ où m et m" sont identiques ou différents et sont un nombre entier compris entre 1 et 3, à un groupe électrodonneur consistant en un ferrocène, une quinone, un ferrocène ou une quinone activé(e) par un ester sélectionné dans le groupe consistant en COON-hydroxyphtalamide, COON-hydroxysuccinimide et COOpentafluorophénol, ledit groupement électrodonneur étant lui-même lié de manière covalente à un antiligand.

**24.** Sonde selon la revendication 23, **caractérisée en ce que** le polymère électroactif est choisi parmi le polypyrrole, le polyacétylène, la polyazine, le poly(p-phénylène), le poly(p-phénylène vinylène), le polypyrène, le polythiophène, le polyfuranne, le polysélénophène, la polypyridazine, le polycarbazole, la polyaniline, les polynucléotides bicaténaires.

**25.** Sonde selon la revendication 23, **caractérisée en ce que** le groupe électrodonneur est choisi parmi le ferrocène, la quinone et les dérivés de ceux-ci.

**26.** Sonde selon la revendication 23, **caractérisée en ce que** l'anti-ligand est lié de manière covalente au groupement électrodonneur, par l'intermédiaire d'un second groupe de liaison.

**27.** Sonde selon la revendication 23, **caractérisée en ce que** l'anti-ligand est lié de manière covalente au polymère électroactif, par l'intermédiaire d'un troisième groupe de liaison.

**28.** Sonde selon l'une quelconque des revendications 24 à 26, **caractérisée en ce que** les second et/ou troisième groupes de liaison relient respectivement, le groupe électrodonneur à l'anti-ligand et l'anti-ligand au polymère électroactif, par l'intermédiaire d'un bras de couplage.

**29.** Sonde selon les revendications 24 et 25, **caractérisée en ce que** le polymère électroactif est le polypyrrole constitué d'au moins deux monomères consistant chacun en un noyau pyrrole, et **en ce que** le groupe électrodonneur est le ferrocène.

**30.** Sonde selon les revendications 23 et 29, **caractérisée en ce que** l'anti-ligand est un polynucléotide sonde, susceptible d'hybrider un polynucléotide cible, dans des conditions d'hybridation appropriées.

**31.** Sonde selon la revendication 30, **caractérisée en ce que** le polynucléotide sonde est lié au ferrocène et au noyau pyrrole dudit monomère au moins.

**32.** Sonde selon la revendication 29, **caractérisée en ce que** le noyau pyrrole est substitué sur le carbone en position 3.

**33.** Sonde selon la revendication 31, **caractérisée en ce que** le polynucléotide sonde est lié sur le carbone en position 1 d'un des noyaux cyclopentadiène du ferrocène.

**34.** Sonde selon la revendication 29, **caractérisée en ce que** le polypyrrole est un copolymère et comprend un monomère dont le noyau pyrrole est substitué par un groupe $-CH_2-COOH$ ou $-CH_2-CH_2OH$.

**35.** Sonde selon la revendication 31, **caractérisée en ce que** le second groupe de liaison, entre le ferrocène et le polynucléotide sonde est le groupe -CO-.

**36.** Sonde selon la revendication 31, **caractérisée en ce que** le troisième groupe de liaison entre le polynucléotide sonde et le noyau pyrrole, est le groupe - $CH_2$-CO-.

**37.** Sonde selon l'une quelconque des revendications 35 à 36, **caractérisée en ce que** les second et/ou troisième groupes de liaison relient, indirectement, respectivement, le ferrocène au polynucléotide sonde et le noyau pyrrole au polynucléotide sonde, par l'intermédiaire d'un bras de couplage.

**38.** Sonde selon la revendication 37, **caractérisée en ce que** le bras de couplage est une chaîne hydrocarbonée saturée ayant au moins deux atomes de carbone, de préférence au moins 2 ou 3 atomes de carbone.

**39.** Sonde selon l'une quelconque des revendications 29 à 38, **caractérisée en ce que** le polynucléotide sonde est fixé au ferrocène et/ou au noyau pyrrole du monomère au moins, par l'intermédiaire respectivement du second et/ou du troisième groupe de liaison et d'au moins une des fonctions aminées du polynucléotide sonde.

**40.** Procédé pour préparer une sonde selon la revendication 28, **caractérisé en ce qu'**il comprend les étapes suivantes :

(a) On dispose d'un polypyrrole homopolymère ou copolymère constitué par au moins deux monomères consistant chacun en un noyau pyrrole, dont au moins un est substitué sur le carbone en position 3 par un polynucléotide sonde,
(b) On obtient le ferrocène comportant sur le carbone en position 1 d'un des noyaux cyclopentadiène, au moins un groupe activé ou activable, et
(c) On met en contact le polypyrrole homopolymère ou copolymère, substitué par un polynucléotide sonde, avec le ferrocène comportant ledit groupe activé ou activable.

**41.** Procédé selon la revendication 40, **caractérisé en ce que** le ou les groupes activés ou activables du ferrocène, identiques ou différents, sont un groupe ester activé ou activable, et de préférence le groupe -CO-[N-hydroxyphtalimide].

**42.** Procédé selon l'une quelconque des revendications 40 ou 41, **caractérisé en ce que** le ou les groupes activés ou activables du ferrocéne, sont fixés sur celui-ci par l'intermédiaire d'un bras de couplage.

**43.** Electrode dont tout ou partie de la surface est revêtue d'une sonde selon l'une quelconque des revendications 23 à 39.

**44.** Utilisation d'un groupe électrodonneur pour augmenter l'électroactivité d'un polymère électroactif sur lequel est fixé un anti-ligand susceptible d'interagir avec un ligand, ledit groupe électrodonneur se trouvant sur le même monomère que l'anti-ligand.

**45.** Procédé de détection d'un ligand dans un échantillon biologique, comportant les étapes consistant à mettre en contact, dans des conditions de réaction appropriées pour l'interaction spécifique anti-ligand / ligand, une sonde électroactive pour la détection et/ou l'identification dudit ligand, et mettre en évidence ou quantifier une différence de potentiel ou une variation de courant entre la sonde avant mise en contact et la sonde après mise en contact, **caractérisée en ce que** ladite sonde est constituée par un polymère homopolymère ou copolymère électroactif d'au moins deux monomères et un anti-ligand, ledit anti-ligand étant lié de manière covalente au polymère électroactif, et ladite sonde comprend en outre au moins un groupe électrodonneur lié de manière covalente à l'anti-ligand, et **en ce qu'**elle présente l'une des structures suivantes :

- le groupement électrodonneur est lié de manière covalente d'une part au polymère électroactif et d'autre part à l'anti-ligand,
- le groupement électrodonneur est lié de manière covalente à l'anti-ligand, ledit anti-ligand étant lui-même lié

de manière covalente au polymère électroactif,

étant exclues les sondes électroactives pour lesquelles ledit polymère électroactif est un polypyrrole lié de manière covalente, par l'intermédiaire d'un groupe $(CH2)_m$ CONH $(CH2)_{m''}$ où m et m" sont identiques ou différents et sont un nombre entier compris entre 1 et 3, à un groupe électrodonneur consistant en un ferrocène, une quinone, un ferrocène ou une quinone activé(e) par un ester sélectionné dans le groupe consistant en COON-hydroxyphtalamide, COON-hydroxysuccinimide et COOpentafluorophénol, ledit groupement électrodonneur étant lui-même lié de manière covalente à un antiligand.

**46.** Procédé selon la revendication 45 **caractérisé en ce que** le polymère électroactif est choisi parmi le polypyrrole, le polyacétylène, la polyazine, le poly(p-phénylène), le poly(p-phénylène vinylène), le polypyrène, le polythiophène, le polyfuranne, le polysélénophène, la polypyridazine, le polycarbazole, la polyaniline, les polynucléotides bicaténaires.

**47.** Procédé selon la revendication 45 **caractérisé en ce que** le groupe électrodonneur est choisi parmi le ferrocène, la quinone et les dérivés de ceux-ci.

**48.** Procédé selon la revendication 45 **caractérisé en ce que** le ligand est une molécule biologique notamment choisie parmi les polynucléotides et les polypeptides.

**49.** Procédé selon la revendication 45, **caractérisée en ce que** le ligand est un polynucléotide.

**50.** Procédé selon la revendication 45 **caractérisé en ce que** le ligand est marqué par un traceur susceptible de générer directement ou indirectement un signal.

**51.** Procédé selon la revendication 45 **caractérisé en ce que** l'anti-ligand est lié de manière covalente au groupement électrodonneur, par l'intermédiaire d'un second groupe de liaison.

**52.** Procédé selon la revendication 51 **caractérisé en ce que** le second groupe de liaison relie le groupe électrodonneur à l'anti-ligand, par l'intermédiaire d'un bras de couplage.

**53.** Procédé selon les revendications 47 et 48 **caractérisé en ce que** le polymère électroactif est le polypyrrole constitué d'au moins deux monomères consistant chacun en un noyau pyrrole, et **en ce que** le groupe électrodonneur est le ferrocène.

**54.** Procédé selon selon les revendications 45 et 53, **caractérisé en ce que** l'anti-ligand est un polynucléotide sonde, susceptible d'hybrider un polynucléotide cible, dans des conditions d'hybridation appropriées.

**55.** Procédé selon la revendication 53 **caractérisé en ce que** le noyau pyrrole est substitué sur le carbone en position 3.

**56.** Procédé selon la revendication 53, **caractérisé en ce que** le polypyrrole est un copolymère et comprend un monomère dont le noyau pyrrole est substitué par un groupe $-CH_2-COOH$ ou $-CH_2-CH_2OH$.

**57.** Procédé selon la revendication 51 **caractérisé en ce que** le second groupe de liaison, entre le ferrocène et le polynucléotide sonde est le groupe -CO-.

**58.** Procédé selon la revendication 57 **caractérisé en ce que** le second groupe de liaison relie le ferrocène au polynucléotide sonde, par l'intermédiaire d'un bras de couplage.

**59.** Procédé selon la revendication 58, **caractérisé en ce que** le bras de couplage est une chaîne hydrocarbonée saturée ayant au moins deux atomes de carbone, de préférence au moins 2 ou 3 atomes de carbone.

**60.** Procédé selon l'une quelconque des revendications 53 à 59, **caractérisé en ce que** le polynucléotide sonde est fixé au ferrocène par l'intermédiaire respectivement du second et/ou du troisième groupe de liaison et d'au moins une des fonctions aminées du polynucléotide sonde.

## Claims

1. An electroactive complex consisting of an electroactive, homopolymer or copolymer, polymer of at least two monomers, an antiligand and a ligand that has specifically interacted with said antiligand, **characterized in that** it further comprises at least one electron-donating group and **in that** it has one of the following structures :

   - the electron-donating group is linked covalently on the one hand to the electroactive polymer and on the other hand to the antiligand, that interacts specifically with the ligand or the antiligand that interacts specifically with the antiligand,
   - the electron-donating group is linked covalently to the antiligand that interacts specifically with the ligand, said antiligand being linked covalently to the electroactive polymer,
   - the electron-donating group is linked covalently to the ligand that interacts specifically with the antiligand, said antiligand being linked covalently to the electroactive polymer,

   the electroactive complexs for which said electroactive polymer is a polypyrrole linked covalently, via a group $(CH_2)_mCONH(CH_2)_{m''}$, where m and m'' are identical or different and integers ranging from 1 to 3, to an electron-donating group consisting of ferrocene, quinine, ferrocene or quinone activated by an ester selected in the group consisting of COON-hydroxyphtalamide, COON-hydroxysuccinimide et COOpentafluorophenol, said electron-donating group being linked covalently to an antiligand wich interacts specifically with an ligand are excluded.

2. The complex as claimed in claim 1, **characterized in that** the electroactive polymer is chosen from polypyrrole, polyacetylene, polyazine, poly(p-phenylene), poly(p-phenylene vinylene), polypyrene, polythiophene, polyfuran, polyselenophene, polypyridazine, polycarbazole, polyaniline and double-stranded polynucleotides.

3. The complex as claimed in claim 1, **characterized in that** the electron-donating group is chosen from ferrocene, quinone and derivatives of these.

4. The complex as claimed in claim 1, **characterized in that** the ligand and the antiligand are biological molecules especially chosen from polynucleotides and polypeptides.

5. The complex as claimed in claim 4, **characterized in that** the ligand and/or the antiligand are labeled by a tracer capable of generating a signal directly or indirectly.

6. The complex as claimed in claim 1, **characterized in that** the antiligand or the ligand are linked covalently to the electron-donating group via a second linking group.

7. The complex as claimed in claim 1, **characterized in that** the antiligand or the ligand are linked covalently to the electroactive polymer via a third linking group.

8. The complex as claimed in either of claims 6 to 7, **characterized in that** the second and/or third linking groups link, respectively, the electron-donating group to the antiligand and the antiligand to the electroactive polymer, via a coupling arm.

9. The complex as claimed in claim 1, **characterized in that** the electron-donating group is linked to the ligand via an inert or biological support.

10. The complex as claimed in claim 9, **characterized in that** the inert support is a polystyrene bead, a magnetic bead or a glass bead.

11. The complex as claimed in claim 9, **characterized in that** the biological support is a cell in which the electron-donating group has been internalized.

12. The complex as claimed in claim 1, **characterized in that** the electroactive polymer is a polypyrrole formed from at least two monomers each consisting of a pyrrole ring and **in that** the electron-donating group is ferrocene.

13. The complex as claimed in claim 12, **characterized in that** the antiligand is a probe polynucleotide and the ligand is a target polynucleotide, at least partly hybridized with said antiligand.

**14.** The complex as claimed in claim 13, **characterized in that** the probe polynucleotide is linked to the ferrocene and to the pyrrole ring of said monomer at least.

**15.** The complex as claimed in claim 12, **characterized in that** the pyrrole ring is substituted on the carbon in the 3 position.

**16.** The complex as claimed in claim 14, **characterized in that** the probe polynucleotide is attached to the carbon in the 1 position of one of the cyclopentadiene rings of the ferrocene.

**17.** The complex as claimed in claim 12, **characterized in that** the polypyrrole is a copolymer and comprises a monomer whose pyrrole ring is substituted with a $-CH_2-COOH$ or $-CH_2-CH_2OH$ group.

**18.** The complex as claimed in claim 14, **characterized in that** the second linking group between the ferrocene and the probe polynucleotide or target polynucleotide is the -CO- group.

**19.** The complex as claimed in claim 14, **characterized in that** the third linking group between the probe polynucleotide or target polynucleotide and the pyrrole ring is the $-CH_2-CO-$ group.

**20.** The complex as claimed in either of claims 18 and 19, **characterized in that** the second and/or third linking groups link, indirectly, the ferrocene to the probe polynucleotide or target polynucleotide, and the pyrrole ring to the probe polynucleotide or target polynucleotide, via a coupling arm.

**21.** The complex as claimed in claim 20, **characterized in that** the coupling arm is a saturated hydrocarbon chain having at least two carbon atoms, preferably at least 2 or 3 carbon atoms.

**22.** The complex as claimed in any one of claims 13 to 21, **characterized in that** the probe polynucleotide or the target polynucleotide is attached to the ferrocene and/or to the pyrrole ring of the monomer at least, via, respectively, the second and/or the third linking groups and via at least one of the amino functional groups of the probe polynucleotide or target polynucleotide.

**23.** An electroactive probe for the detection and/or the identification of a ligand, consisting of an electroactive, homopolymer or copolymer, polymer of at least two monomers and an antiligand, **characterized in that** said electroactive probe further comprises at least one electron-donating group and **in that** said electroactive probe has one of the two following structures :

- the electron-donating group is linked covalently on the one hand to the electroactive polymer and on the other hand to the antiligand,
- the electron-donating group is linked covalently to the antiligand, said antiligand being linked covalently to the electroactive polymer,

the electroactive probes for which the electroactive polymer is a polypyrrole linked covalently, via a group $(CH_2)_mCONH(CH_2)_{m''}$, where m and m" are identical or different and integers ranging from 1 to 3, to an electron-donating group consisting of ferrocene, quinine, ferrocene or quinone activated by an ester selected in the group consisting of COON-hydroxyphtalamide, COON-hydroxysuccinimide et COOpentafluorophenol, said electron-donating group being linked covalently to an antiligand wich interacts specifically with an ligand are excluded.

**24.** The probe as claimed in claim 23, **characterized in that** the electroactive polymer is chosen from polypyrrole, polyacetylene, polyazine, poly(p-phenylene), poly(p-phenylene vinylene), polypyrene, polythiophene, polyfuran, polyselenophene, polypyridazine, polycarbazole, polyaniline and double-stranded polynucleotides.

**25.** The probe as claimed in claim 23, **characterized in that** the electron-donating group is chosen from ferrocene, quinone and derivatives of these.

**26.** The probe as claimed in claim 23, **characterized in that** the antiligand is linked covalently to the electron-donating group via a second linking group.

**27.** The probe as claimed in claim 23, **characterized in that** the antiligand is linked covalently to the electroactive polymer via a third linking group.

**28.** The probe as claimed in any one of claims 24 to 26, **characterized in that** the second and/or third linking groups link, respectively, the electron-donating group to the antiligand, and the antiligand to the electroactive polymer, via a coupling arm.

**29.** The probe as claimed in claims 24 and 25, **characterized in that** the electroactive polymer is polypyrrole formed from at least two monomers each consisting of a pyrrole ring and **in that** the electron-donating group is ferrocene.

**30.** The probe as claimed in claims 23 and 29, **characterized in that** the antiligand is a probe polynucleotide capable of hybridizing a target polynucleotide under appropriate hybridization conditions.

**31.** The probe as claimed in claim 30, **characterized in that** the probe polynucleotide is linked to the ferrocene and to the pyrrole ring of said monomer at least.

**32.** The probe as claimed in claim 29, **characterized in that** the pyrrole ring is substituted on the carbon in the 3 position.

**33.** The probe as claimed in claim 31, **characterized in that** the probe polynucleotide is attached to the carbon in the 1 position of one of the cyclopentadiene rings of the ferrocene.

**34.** The probe as claimed in claim 29, **characterized in that** the polypyrrole is a copolymer and comprises a monomer whose pyrrole ring is substituted with a $-CH_2-COOH$ or $-CH_2-CH_2OH$ group.

**35.** The probe as claimed in claim 31, **characterized in that** the second linking group between the ferrocene and the probe polynucleotide is the -CO- group.

**36.** The probe as claimed in claim 31, **characterized in that** the third linking group between the probe polynucleotide and the pyrrole ring is the $-CH_2-CO-$ group.

**37.** The probe as claimed in either of claims 35 and 36, **characterized in that** the second and/or third linking groups link, indirectly, the ferrocene to the probe polynucleotide, and the pyrrole ring to the probe polynucleotide, respectively, via a coupling arm.

**38.** The probe as claimed in claim 37, **characterized in that** the coupling arm is a saturated hydrocarbon chain having at least two carbon atoms, preferably at least 2 or 3 carbon atoms.

**39.** The probe as claimed in any one of claims 29 to 38, **characterized in that** the probe polynucleotide is attached to the ferrocene and/or to the pyrrole ring of the monomer at least, via, respectively, the second and/or the third linking group and via at least one of the amino functional groups of the probe polynucleotide.

**40.** A method for preparing a probe as claimed in claim 28, **characterized in that** it comprises the following steps:

(a) a homopolymer or copolymer polypyrrole formed by at least two monomers each consisting of a pyrrole ring, at least one of which is substituted on the carbon in the 3 position with a probe polynucleotide, is obtained;
(b) ferrocene having, on the carbon in the 1 position of one of the cyclopentadiene rings, at least one activated or activatable group is obtained; and
(c) the homopolymer or copolymer polypyrrole, substituted with a probe polynucleotide, is brought into contact with the ferrocene having said activated or activatable group.

**41.** The method as claimed in claim 40, **characterized in that** the activated or activatable group or groups of the ferrocene, which may be identical or different, are an activated or activatable ester group and preferably a -CO-[N-hydroxyphthalimide] group.

**42.** The method as claimed in either of claims 40 and 41, **characterized in that** the activated or activatable group or groups of the ferrocene are attached to the latter via a coupling arm.

**43.** An electrode, all or part of the surface of which is coated with a probe as claimed in any one of claims 23 to 39.

**44.** The use of an electron-donating group to increase the electroactivity of an electroactive polymer to which an antiligand capable of interacting with a ligand is attached, said electron-donating group being on the same monomer as the

antiligand.

**45.** A method of detecting a ligand in a biological specimen, comprising the steps consisting of bringing into contact under appropriate reaction conditions for the specific ligand/antiligand interaction, an electroactive probe for the detection and or the identification of said ligand, and demonstrating or quantifying a difference in poytential or a variation in current between the probe before contacting and the probe after contacting, **characterized in that** said electroactive probe is constituted of an electroactive, homopolymer or copolymer, polymer of at least two monomers and an antiligand capable of interacting specifically with electroactive polymer, said probe furthermore including at least one electron-donating group linked covalently to the antiligand, and **in that** said electroactive probe has one of the following structures :

- the electron-donating group is linked covalently on the one hand to the electroactive polymer and on the other hand to the antiligand,
- the electron-donating group is linked covalently to the antiligand, said antiligand being linked covalently to the electroactive polymer,

the electroactive probes for which the electroactive polymer is a polypyrrole linked covalently, via a group $(CH_2)_mCONH(CH_2)_{m''}$, where m and m" are identical or different and integers ranging from 1 to 3, to an electron-donating group consisting of ferrocene, quinine, ferrocene or quinone activated by an ester selected in the group consisting of COON-hydroxyphtalamide, COON-hydroxysuccinimide et COOpentafluorophenol, said electron-donating group being linked covalently to an antiligand wich interacts specifically with an ligand are excluded.

**46.** The method as claimed in claim 45, **characterized in that** the electroactive polymer is chosen from polypyrrole, polyacetylene, polyazine, poly(p-phenylene), poly(p-phenylene vinylene), polypyrene, polythiophene, polyfuran, polyselenophene, polypyridazine, polycarbazole, polyaniline and double-stranded polynucleotides.

**47.** The method as claimed in claim 45, **characterized in that** the electron-donating group is chosen from ferrocene, quinone and derivatives of these.

**48.** The method as claimed in claim 45, **characterized in that** the ligand is a biological molecule especially chosen from polynucleotides and polypeptides.

**49.** The method as claimed in claim 45, **characterized in that** the ligand is a polynucleotide.

**50.** The method as claimed in claim 45, **characterized in that** the ligand is labeled by a tracer capable of generating a signal directly or indirectly.

**51.** The method as claimed in claim 45, **characterized in that** the antiligand is linked covalently to the electron-donating group via a second linking group.

**52.** The method as claimed in claim 51, **characterized in that** the second linking group links, respectively, the electron-donating group to the the antiligand, via a coupling arm.

**53.** The method as claimed in claim 51, **characterized in that** the electroactive polymer is the polypyrrole formed from at least two monomers each consisting of a pyrrole ring and **in that** the electron-donating group is ferrocene.

**54.** The method as claimed in claims 45 to 53, **characterized in that** the antiligand is a probe polynucleotide capable of hybridizing a target polynucleotide under appropriate hybridization conditions.

**55.** The method as claimed in claim 53, **characterized in that** the pyrrole ring is substituted on the carbon in the 3 position.

**56.** The method as claimed in claim 53, **characterized in that** the polypyrrole is a copolymer and comprises a monomer whose pyrrole ring is substituted with a $-CH_2-COOH$ or $-CH_2-CH_2OH$ group.

**57.** The method as claimed in claim 51, **characterized in that** the second linking group between the ferrocene and the probe polynucleotide is the -CO- group.

**58.** The method as claimed in claim 57, **characterized in that** the second links the ferrocene to the pyrrole probe

polynucleotide, respectively, via a coupling arm.

**59.** The method as claimed in claim 58, **characterized in that** the coupling arm is a saturated hydrocarbon chain having at least two carbon atoms, preferably at least 2 or 3 carbon atoms.

**60.** The probe as claimed in any one of claims 53 to 59, **characterized in that** the probe polynucleotide is attached to the ferrocene via, respectively, the second and/or the third linking group and via at least one of the amino functional groups of the probe polynucleotide.

**Patentansprüche**

**1.** Elektroaktiver Komplex, der aus Folgendem gebildet wird, nämlich einem elektroaktiven homopolymeren oder copolymeren Polymer aus zumindest zwei Monomeren, einem Anti-Liganden und einem Liganden, der mit dem Anti-Liganden spezifisch interagiert hat, **dadurch gekennzeichnet, dass** er ferner zumindest eine Elektronendonorgruppe aufweist und dass er eine der folgenden drei Strukturen aufweist:

- die Elektronendonorgruppe ist auf kovalente Weise auf der einen Seite an das elektroaktive Polymer und auf der anderen Seite an den Anti-Liganden, der spezifisch mit dem Liganden interagiert oder an den Liganden gebunden, der spezifisch mit dem Anti-Liganden interagiert,
- die Elektronendonorgruppe ist auf kovalente Weise an den Anti-Liganden gebunden, der spezifisch mit dem Liganden interagiert, wobei der Anti-Ligand wiederum auf kovalente Weise an das elektroaktive Polymer gebunden ist,
- die Elektronendonorgruppe ist auf kovalente Weise an den Liganden gebunden, der spezifisch mit dem Anti-Liganden interagiert, wobei der Anti-Ligand auf kovalente Weise an das elektroaktive Polymer gebunden ist,

wobei die elektroaktiven Komplexe ausgeschlossen sind, in denen das elektroaktive Polymer ein Polypyrrol ist, das auf kovalente Weise über eine $(CH2)_mCONH(CH2)_{m''}$-Gruppe, in der m und m'' gleich oder unterschiedlich und eine ganze Zahl von 1 bis 3 sind, an eine Elektronendonorgruppe gebunden ist, die aus Folgendem besteht, nämlich einem Ferrocen, einem Chinon, oder einem durch einen Ester, der ausgewählt ist aus der Gruppe bestehend aus COON-Hydroxyphthalamid, COON-Hydroxysuccinimid und COO-Pentafluorphenol, aktivierten Ferrocen oder Chinon, wobei die Elektronendonorgruppe wiederum auf kovalente Weise an einen Anti-Liganden gebunden ist, der spezifisch mit einem Liganden interagiert.

**2.** Komplex nach Anspruch 1, **dadurch gekennzeichnet, dass** das elektroaktive Polymer ausgewählt ist aus Polypyrrol, Polyacetylen, Polyazin, Poly(p-phenylen), Poly(p-phenylenvinylen), Polypyren, Polythiophen, Polyfuran, Polyselenophen, Polypyridazin, Polycarbazol, Polyanilin und den doppelsträngigen Polynucleotiden.

**3.** Komplex nach Anspruch 1, **dadurch gekennzeichnet, dass** die Elektronendonorgruppe ausgewählt ist aus Ferrocen, Chinon und deren Derivaten.

**4.** Komplex nach Anspruch 1, **dadurch gekennzeichnet, dass** der Ligand und der Anti-Ligand biologische Moleküle sind, die insbesondere ausgewählt sind aus den Polynucleotiden und den Polypeptiden.

**5.** Komplex nach Anspruch 4, **dadurch gekennzeichnet, dass** der Ligand und/oder der Anti-Ligand mit einem Tracer markiert sind, der dazu in der Lage ist, direkt oder indirekt ein Signal zu erzeugen.

**6.** Komplex nach Anspruch 1, **dadurch gekennzeichnet, dass** der Anti-Ligand oder der Ligand über eine zweite Verbindungsgruppe auf kovalente Weise an die Elektronendonorgruppe gebunden ist.

**7.** Komplex nach Anspruch 1, **dadurch gekennzeichnet, dass** der Anti-Ligand oder der Ligand über eine dritte Verbindungsgruppe auf kovalente Weise an das elektroaktive Polymer gebunden ist.

**8.** Komplex nach einem der Ansprüche 6 bis 7, **dadurch gekennzeichnet, dass** die zweite und/oder dritte Verbindungsgruppe über einen Kupplungsarm die Elektronendonorgruppe mit dem Anti-Liganden bzw. den Anti-Liganden mit dem elektroaktiven Polymer verbinden.

**9.** Komplex nach Anspruch 1, **dadurch gekennzeichnet, dass** die Elektronendonorgruppe über einen inerten oder

biologischen Träger mit dem Liganden verbunden ist.

**10.** Komplex nach Anspruch 9, **dadurch gekennzeichnet, dass** der inerte Träger ein Polystyrenkügelchen, ein magnetisches Kügelchen oder ein Glaskügelchen ist.

**11.** Komplex nach Anspruch 9, **dadurch gekennzeichnet, dass** der biologische Träger eine Zelle ist, in der die Elektronendonorgruppe aufgenommen ist.

**12.** Komplex nach Anspruch 1, **dadurch gekennzeichnet, dass** das elektroaktive Polymer ein Polypyrrol ist, das aus zumindest zwei Monomeren besteht, die je einen Pyrrolring aufweisen und dass die Elektronendonorgruppe Ferrocen ist.

**13.** Komplex nach Anspruch 12, **dadurch gekennzeichnet, dass** der Anti-Ligand ein Sondenpolynucleotid ist und dass der Ligand ein Zielpolynucleotid ist, das zumindest zum Teil mit dem Anti-Liganden hybridisiert ist.

**14.** Komplex nach Anspruch 13, **dadurch gekennzeichnet, dass** das Sondenpolynucleotid zumindest mit dem Ferrocen und dem Pyrrolring des Monomers verbunden ist.

**15.** Komplex nach Anspruch 12, **dadurch gekennzeichnet, dass** der Pyrrolring am Kohlenstoff in 3-Position substituiert ist.

**16.** Komplex nach Anspruch 14, **dadurch gekennzeichnet, dass** das Sondenpolynucleotid am Kohlenstoff in 1-Position eines der Cyclopentadienringe des Ferrocens angebracht ist.

**17.** Komplex nach Anspruch 12, **dadurch gekennzeichnet, dass** das Polypyrrol ein Copolymer ist und ein Monomer aufweist, in dem der Pyrrolring mit einer $-CH_2-COOH-$ oder einer - $CH_2-CH_2OH$-Gruppe substituiert ist.

**18.** Komplex nach Anspruch 14, **dadurch gekennzeichnet, dass** die zweite Verbindungsgruppe zwischen dem Ferrocen und dem Sondenpolynucleotid oder dem Zielpolynucleotid eine-CO-Gruppe ist.

**19.** Komplex nach Anspruch 14, **dadurch gekennzeichnet, dass** die dritte Verbindungsgruppe zwischen dem Sondenpolynucleotid oder dem Zielpolynucleotid und dem Pyrrolring eine - $CH_2$-CO-Gruppe ist.

**20.** Komplex nach einem der Ansprüche 18 bis 19, **dadurch gekennzeichnet, dass** die zweite und/oder dritte Verbindungsgruppe über einen Kupplungsarm auf indirekte Weise das Ferrocen mit dem Sondenpolynucleotid oder dem Zielpolynucleotid bzw. den Pyrrolring mit dem Sondenpolynucleotid oder dem Zielpolynucleotid verbinden.

**21.** Komplex nach Anspruch 20, **dadurch gekennzeichnet, dass** der Kupplungsarm eine gesättigte Kohlenwasserstoffkette ist, die zumindest zwei Kohlenstoffatome, bevorzugterweise zumindest zwei oder drei Kohlenstoffatome aufweist.

**22.** Komplex nach einem der Ansprüche 13 bis 21, **dadurch gekennzeichnet, dass** das Sondenpolynucleotid oder das Zielpolynucleotid über die zweite und/oder dritte Verbindungsgruppe und zumindest eine der Aminfunktionen des Sondenpolynucleotids oder des Zielpolynucleotids mit dem Ferrocen und/oder dem Pyrrolring des Monomers verbunden ist.

**23.** Elektroaktive Sonde zur Detektion und/oder Identifikation eines Liganden, die aus einem elektroaktiven homopolymeren oder copolymeren Polymer aus zumindest zwei Monomeren und einem Anti-Liganden gebildet wird, **dadurch gekennzeichnet, dass** sie ferner zumindest eine Elektronendonorgruppe aufweist und dass sie eine der folgenden beiden Strukturen aufweist:

- die Elektronendonorgruppe ist auf kovalente Weise an das elektroaktive Polymer und auf der anderen Seite an den Anti-Liganden gebunden,
- die Elektronendonorgruppe ist auf kovalente Weise auf der einen Seite an den Anti-Liganden gebunden, wobei der Anti-Ligand wiederum auf kovalente Weise an das elektroaktive Polymer gebunden ist,

wobei die elektroaktiven Sonden ausgeschlossen sind, in denen das elektroaktive Polymer ein Polypyrrol ist, das auf kovalente Weise über eine $(CH2)_mCONH(CH2)_{m''}$-Gruppe, in der m und m" gleich oder unterschiedlich und eine

ganze Zahl von 1 bis 3 sind, an eine Elektronendonorgruppe gebunden ist, die aus Folgendem besteht, nämlich einem Ferrocen, einem Chinon oder einem durch einen Ester, der ausgewählt ist aus der Gruppe bestehend aus COON-Hydroxyphthalamid, COON-Hydroxysuccinimid und COO-Pentafluorphenol, aktivierten Ferrocen oder Chinon, wobei die Elektronendonorgruppe wiederum auf kovalente Weise an einen Anti-Liganden gebunden ist.

**24.** Sonde nach Anspruch 23, **dadurch gekennzeichnet, dass** das elektroaktive Polymer ausgewählt ist aus Polypyrrol, Polyacetylen, Polyazin, Poly(p-phenylen), Poly(p-phenylenvinylen), Polypyren, Polythiophen, Polyfuran, Polyselenophen, Polypyridazin, Polycarbazol, Polyanilin und den doppelsträngigen Polynucleotiden.

**25.** Sonde nach Anspruch 23, **dadurch gekennzeichnet, dass** die Elektronendonorgruppe ausgewählt ist aus Ferrocen, Chinon und deren Derivaten.

**26.** Sonde nach Anspruch 23, **dadurch gekennzeichnet, dass** der Anti-Ligand über eine zweite Verbindungsgruppe auf kovalente Weise an die Elektronendonorgruppe gebunden ist.

**27.** Sonde nach Anspruch 23, **dadurch gekennzeichnet, dass** der Anti-Ligand über eine dritte Verbindungsgruppe auf kovalente Weise an das elektroaktive Polymer gebunden ist.

**28.** Sonde nach einem der Ansprüche 24 bis 26, **dadurch gekennzeichnet, dass** die zweite und/oder dritte Verbindungsgruppe über einen Kupplungsarm die Elektronendonorgruppe mit dem Anti-Liganden bzw. den Anti-Liganden mit dem elektroaktiven Polymer verbinden.

**29.** Sonde nach den Ansprüchen 24 und 25, **dadurch gekennzeichnet, dass** das elektroaktive Polymer ein Polypyrrol ist, das aus zumindest zwei Monomeren besteht, die je einen Pyrrolring aufweisen, und dass die Elektronendonorgruppe Ferrocen ist.

**30.** Sonde nach den Ansprüchen 23 und 29, **dadurch gekennzeichnet, dass** der Anti-Ligand ein Sondenpolynucleotid ist, das unter geeigneten Hybridisierungsbedingungen in der Lage ist, mit einem Zielpolynucleotid zu hybridisieren.

**31.** Sonde nach Anspruch 30, **dadurch gekennzeichnet, dass** das Sondenpolynucleotid zumindest mit dem Ferrocen und dem Pyrrolring des Monomers verbunden ist.

**32.** Sonde nach Anspruch 29, **dadurch gekennzeichnet, dass** der Pyrrolring am Kohlenstoff in 3-Position substituiert ist.

**33.** Sonde nach Anspruch 31, **dadurch gekennzeichnet, dass** das Sondenpolynucleotid am Kohlenstoff in 1-Position eines der Cyclopentadienringe des Ferrocens angebracht ist.

**34.** Sonde nach Anspruch 29, **dadurch gekennzeichnet, dass** das Polypyrrol ein Copolymer ist und ein Monomer aufweist, in dem der Pyrrolring mit einer $-CH_2-COOH-$ oder einer $-CH_2-CH_2OH$-Gruppe substituiert ist.

**35.** Sonde nach Anspruch 31, **dadurch gekennzeichnet, dass** die zweite Verbindungsgruppe zwischen dem Ferrocen und dem Sondenpolynucleotid eine -CO-Gruppe ist.

**36.** Sonde nach Anspruch 31, **dadurch gekennzeichnet, dass** die dritte Verbindungsgruppe zwischen dem Sondenpolynucleotid und dem Pyrrolring eine $-CH_2-CO$-Gruppe ist.

**37.** Sonde nach einem der Ansprüche 35 bis 36, **dadurch gekennzeichnet, dass** die zweite und/oder dritte Verbindungsgruppe über einen Kupplungsarm auf indirekte Weise das Ferrocen mit dem Sondenpolynucleotid bzw. den Pyrrolring mit dem Sondenpolynucleotid verbinden.

**38.** Sonde nach Anspruch 37, **dadurch gekennzeichnet, dass** der Kupplungsarm eine gesättigte Kohlenwasserstoffkette ist, die zumindest zwei Kohlenstoffatome, vorzugsweise zumindest zwei oder drei Kohlenstoffatome aufweist.

**39.** Sonde nach einem der Ansprüche 29 bis 38, **dadurch gekennzeichnet, dass** das Sondenpolynucleotid über die zweite und/oder dritte Verbindungsgruppe und zumindest eine der Aminfunktionen des Sondenpolynucleotids mit dem Ferrocen und/oder dem Pyrrolring des Monomers verbunden ist.

**40.** Verfahren zum Herstellen einer Sonde nach Anspruch 28, **dadurch gekennzeichnet, dass** es die folgenden Schritte aufweist:

(a) Bereitstellen eines homopolymeren oder copolymeren Polypyrrol das aus zumindest zwei Monomeren besteht, die je einen Pyrrolring aufweisen, von denen zumindest einer am Kohlenstoff in 3-Position mit einem Sondenpolynucleotid substituiert ist,
(b) Bereitstellen eines Ferrocens, das am Kohlenstoff in 1-Position eines der Cyclopentadienringe zumindest eine aktivierte oder aktivierbare Gruppe aufweist, und
(c) In-Kontakt-Bringen des mit einem Sondenpolynucleotid substituierten homopolymeren oder copolymeren Polypyrrol mit dem Ferrocen, das die aktivierte oder aktivierbare Gruppe trägt.

**41.** Verfahren nach Anspruch 40, **dadurch gekennzeichnet, dass** die aktivierten oder aktivierbaren Gruppen des Ferrocens gleich oder unterschiedlich und eine aktivierte oder aktivierbare Estergruppe, vorzugsweise eine -CO-[N-hydroxyphthalimid]-Gruppe sind.

**42.** Verfahren nach Anspruch 40 oder 41, **dadurch gekennzeichnet, dass** die aktivierten oder aktivierbaren Gruppen des Ferrocens über einen Kupplungsarm an dieses gebunden sind.

**43.** Elektrode, von der ein Teil oder die Gesamtheit der Oberfläche mit einer Sonde nach einem der Ansprüche 23 bis 39 beschichtet ist.

**44.** Verwendung einer Elektronendonorgruppe zum Verstärken der Elektroaktivität eines elektroaktiven Polymers, an dem ein Anti-Ligand angebracht ist, der in der Lage ist, mit einem Liganden zu interagieren, wobei sich die Elektronendonorgruppe am gleichen Monomer wie der Anti-Ligand befindet.

**45.** Verfahren zur Detektion eines Liganden in einer biologischen Probe, das die folgenden Schritte aufweist, nämlich In-Kontakt-Bringen einer elektroaktiven Sonde zur Detektion und/oder Identifikation des Liganden unter geeigneten Reaktionsbedingungen für eine spezifische Anti-Ligand/Ligand-Interaktion und Aufzeigen oder Quantifizieren einer Potenzialdifferenz oder einer Änderung im Strom zwischen der Sonde, bevor sie in Kontakt gebracht wurde, und der Sonde, nachdem sie in Kontakt gebracht wurde, **dadurch gekennzeichnet, dass** die Sonde aus einem elektroaktiven homopolymeren oder copolymeren Polymer aus zumindest zwei Monomeren und einem Anti-Liganden gebildet wird, wobei der Anti-Ligand auf kovalente Weise an das elektroaktive Polymer gebunden ist, und dass die Sonde ferner zumindest eine Elektronendonorgruppe aufweist, die auf kovalente Weise an den Anti-Liganden gebunden ist, und dass sie eine der folgenden Strukturen aufweist:

- die Elektronendonorgruppe ist auf kovalente Weise auf der einen Seite an das elektroaktive Polymer und auf der anderen Seite an den Anti-Liganden gebunden,
- die Elektronendonorgruppe ist auf kovalente Weise an den Anti-Liganden gebunden, wobei der Anti-Ligand wiederum auf kovalente Weise an das elektroaktive Polymer gebunden ist,

wobei die elektroaktiven Sonden ausgeschlossen sind, in denen das elektroaktive Polymer ein Polypyrrol ist, das auf kovalente Weise über eine $(CH2)_{m}CONH(CH2)_{m''}$-Gruppe, in der m und m'' gleich oder unterschiedlich und eine ganze Zahl von 1 bis 3 sind, an eine Elektronendonorgruppe gebunden ist, die aus Folgendem besteht, nämlich einem Ferrocen, einem Chinon oder einem durch einen Ester, der ausgewählt ist aus der Gruppe bestehend aus COON-Hydroxyphthalamid, COON-Hydroxysuccinimid und COO-Pentafluorphenol, aktivierten Ferrocen oder Chinon, wobei die Elektronendonorgruppe wiederum auf kovalente Weise an einen Anti-Liganden gebunden ist.

**46.** Verfahren nach Anspruch 45, **dadurch gekennzeichnet, dass** das elektroaktive Polymer ausgewählt ist aus Polypyrrol, Polyacetylen, Polyazin, Poly(p-phenylen), Poly(p-phenylenvinylen), Polypyren, Polythiophen, Polyfuran, Polyselenophen, Polypyridazin, Polycarbazol, Polyanilin und den doppelsträngigensträngigen Polynucleotiden.

**47.** Verfahren nach Anspruch 45, **dadurch gekennzeichnet, dass** die Elektronendonorgruppe ausgewählt ist aus Ferrocen, Chinon und deren Derivaten.

**48.** Verfahren nach Anspruch 45, **dadurch gekennzeichnet, dass** der Ligand ein biologisches Molekül ist, das insbesondere ausgewählt ist aus den Polynucleotiden und den Polypeptiden.

**49.** Verfahren nach Anspruch 45, **dadurch gekennzeichnet, dass** der Ligand ein Polynucleotid ist.

**50.** Verfahren nach Anspruch 45, **dadurch gekennzeichnet, dass** der Ligand mit einem Tracer markiert ist, der in der Lage ist, direkt oder indirekt ein Signal zu erzeugen.

**51.** Verfahren nach Anspruch 45, **dadurch gekennzeichnet, dass** der Anti-Ligand über eine zweite Verbindungsgruppe auf kovalente Weise an die Elektronendonorgruppe gebunden ist.

**52.** Verfahren nach Anspruch 51, **dadurch gekennzeichnet, dass** die zweite Verbindungsgruppe über einen Kupplungsarm die Elektronendonorgruppe mit dem Anti-Liganden verbindet.

**53.** Verfahren nach den Ansprüchen 47 und 48, **dadurch gekennzeichnet, dass** das elektroaktive Polymer ein Polypyrrol ist, das aus zumindest zwei Monomeren besteht, die je einen Pyrrolring aufweisen, und dass die Elektronendonorgruppe Ferrocen ist.

**54.** Verfahren nach den Ansprüchen 45 und 53, **dadurch gekennzeichnet, dass** der Anti-Ligand ein Sondenpolynucleotid ist, das unter geeigneten Hybridisierungsbedingungen in der Lage ist, mit einem Zielpolynucleotid zu hybridisieren.

**55.** Verfahren nach Anspruch 53, **dadurch gekennzeichnet, dass** der Pyrrolring am Kohlenstoff in 3-Position substituiert ist.

**56.** Verfahren nach Anspruch 53, **dadurch gekennzeichnet, dass** das Polypyrrol ein Copolymer ist und ein Monomer aufweist, in dem der Pyrrolring mit einer $-CH_2-COOH-$ oder einer $CH_2-CH_2OH$-Gruppe substituiert ist.

**57.** Verfahren nach Anspruch 51, **dadurch gekennzeichnet, dass** die zweite Verbindungsgruppe zwischen dem Ferrocen und dem Sondenpolynucleotid eine -CO-Gruppe ist.

**58.** Verfahren nach Anspruch 57, **dadurch gekennzeichnet, dass** die zweite Verbindungsgruppe über einen Kupplungsarm das Ferrocen mit dem Sondenpolynucleotid verbindet.

**59.** Verfahren nach Anspruch 58, **dadurch gekennzeichnet, dass** der Kupplungsarm eine gesättigte Kohlenwasserstoffkette ist, die zumindest zwei Kohlenstoffatome, vorzugsweise zumindest zwei oder drei Kohlenstoffatome aufweist.

**60.** Verfahren nach einem der Ansprüche 53 bis 59, **dadurch gekennzeichnet, dass** das Sondenpolynucleotid über die zweite und/oder dritte Verbindungsgruppe und zumindest eine der Aminfunktionen des Sondenpolynucleotid mit dem Ferrocen verbunden ist.

FIG 1

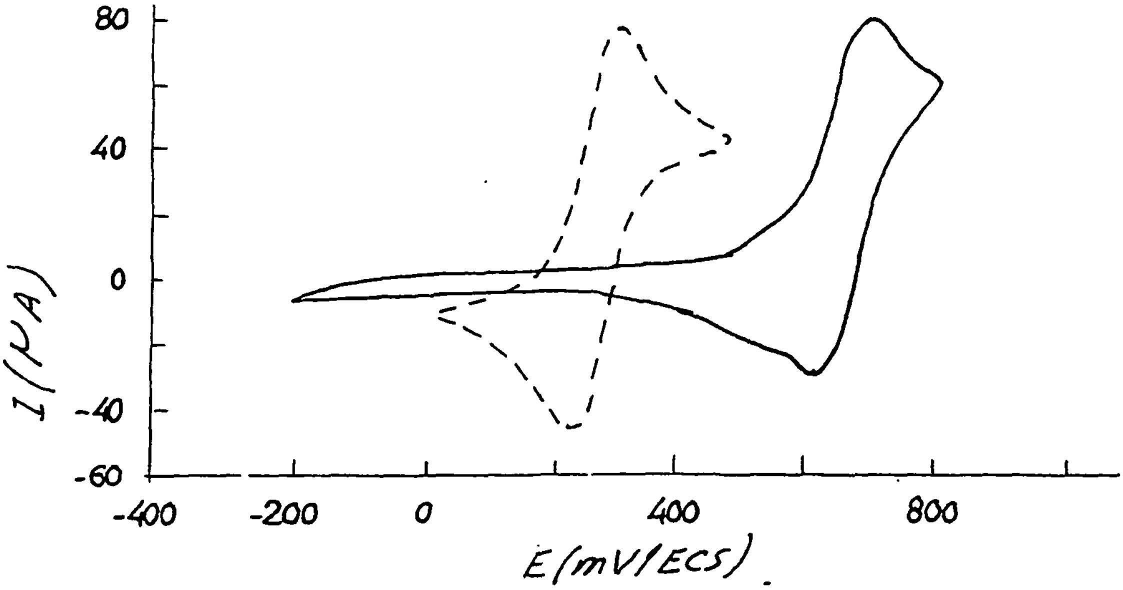
80
40
0
-40
-60
I(μA)
-400
-200
0
400
800
E(mV/ECS)

FIG 2

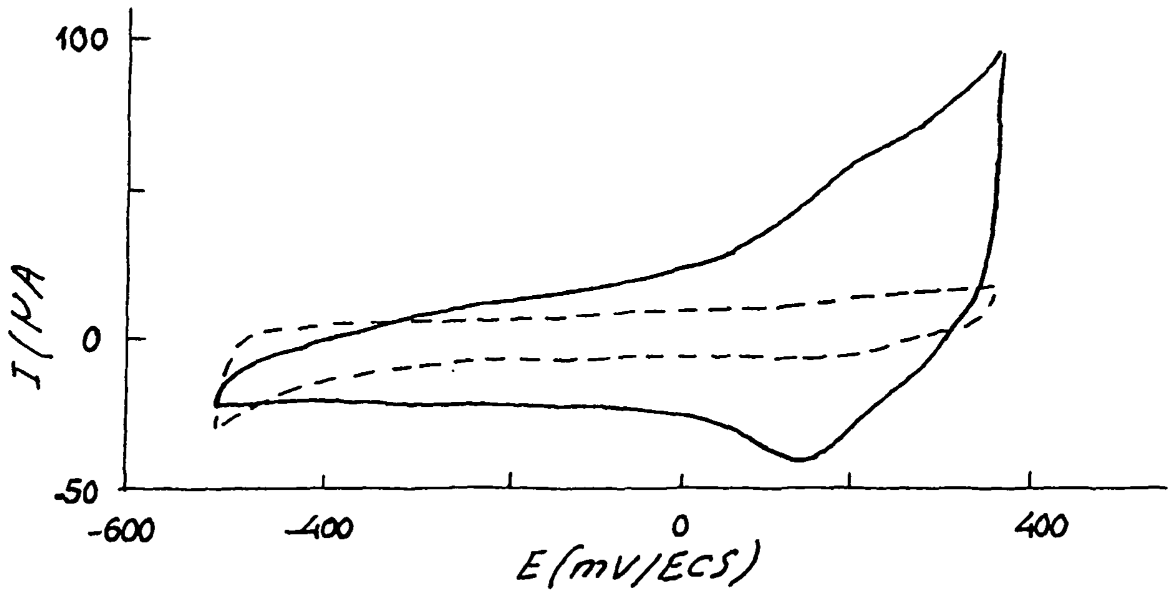
100
0
-50
I(μA)
-600
-400
0
400
E(mV/ECS)

FIG 3

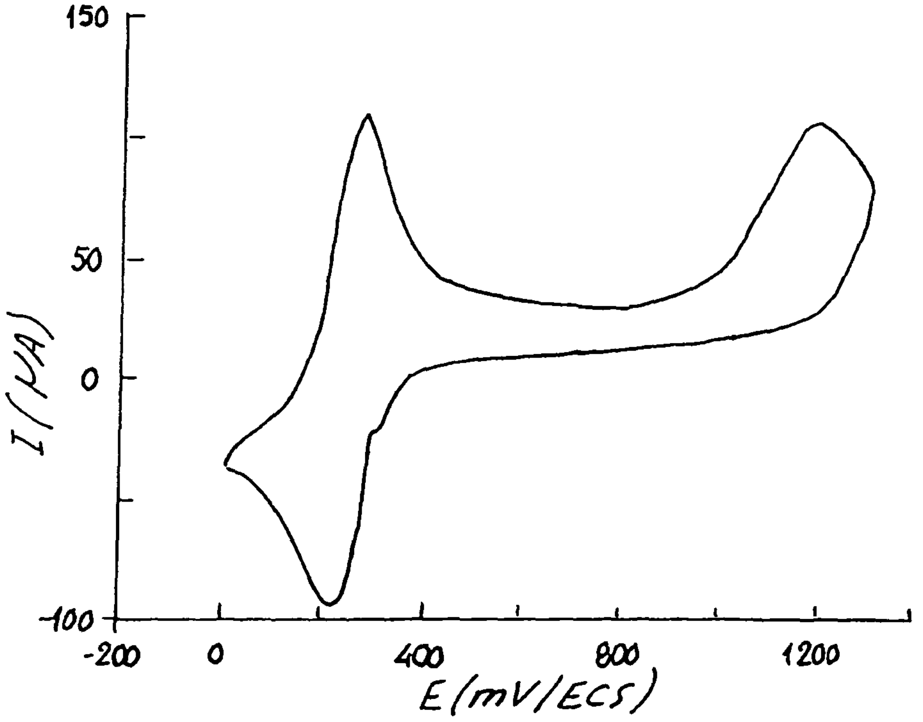
150
50
0
-100
I (μA)
-200
0
400
800
1200
E (mV/ECS)

FIG 4

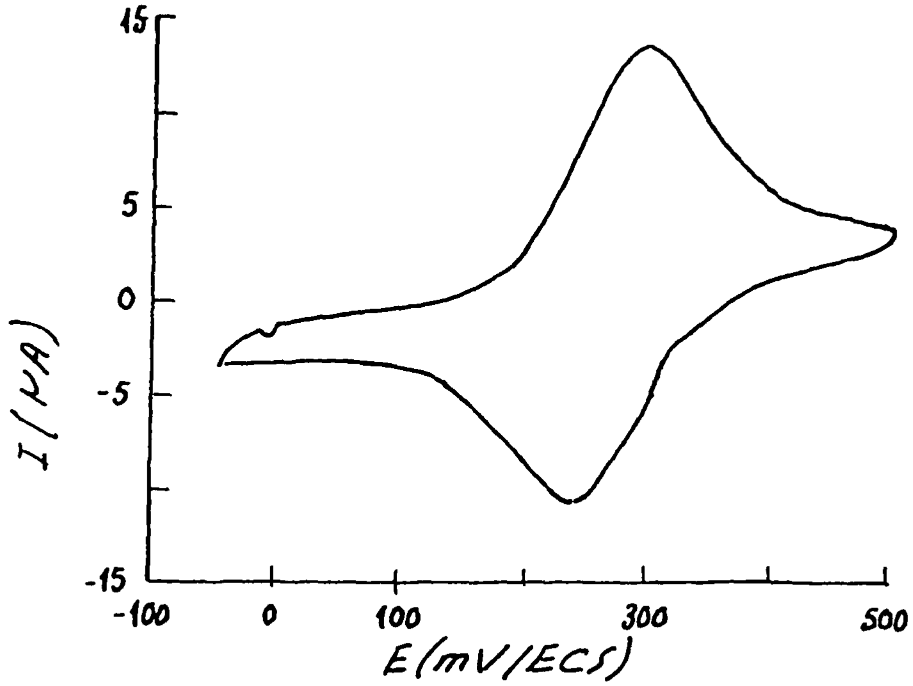
15
5
0
-5
-15
I (μA)
-100
0
100
300
500
E (mV/ECS)

FIG 5

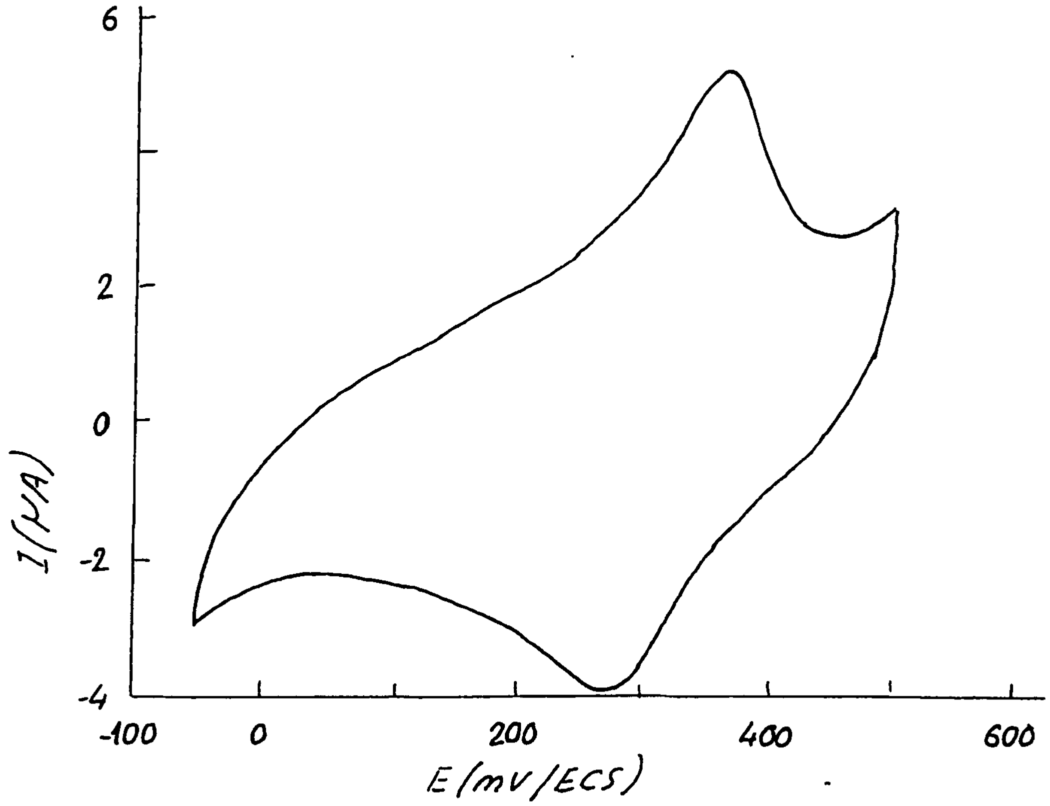
6
2
0
-2
-4
I (μA)
-100
0
200
400
600
E (mV/ECS)

FIG 6

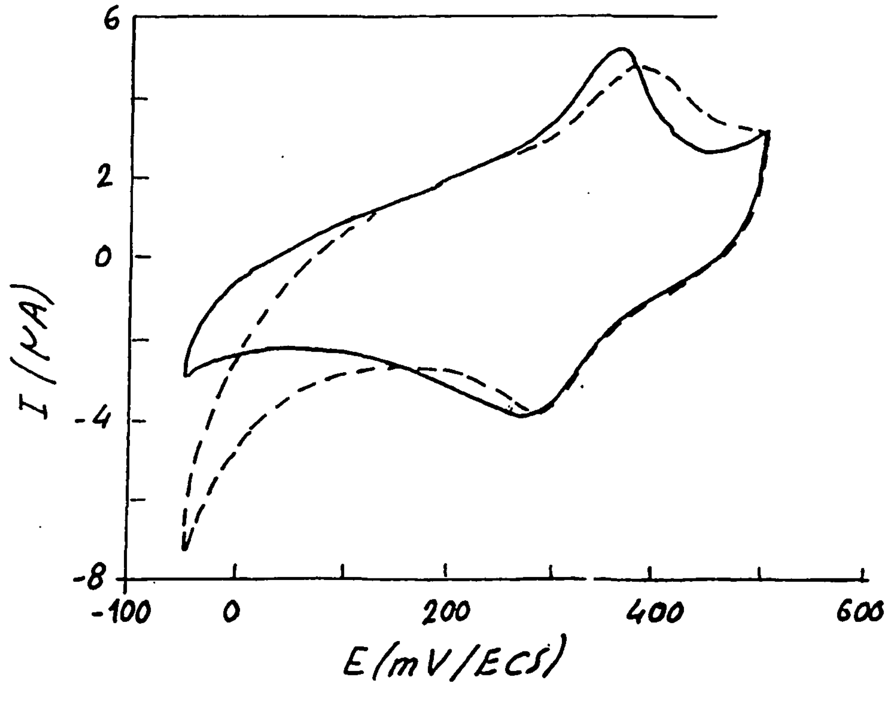
6
2
0
-4
-8
I (μA)
-100
0
200
400
600
E (mV/ECS)

FIG 8

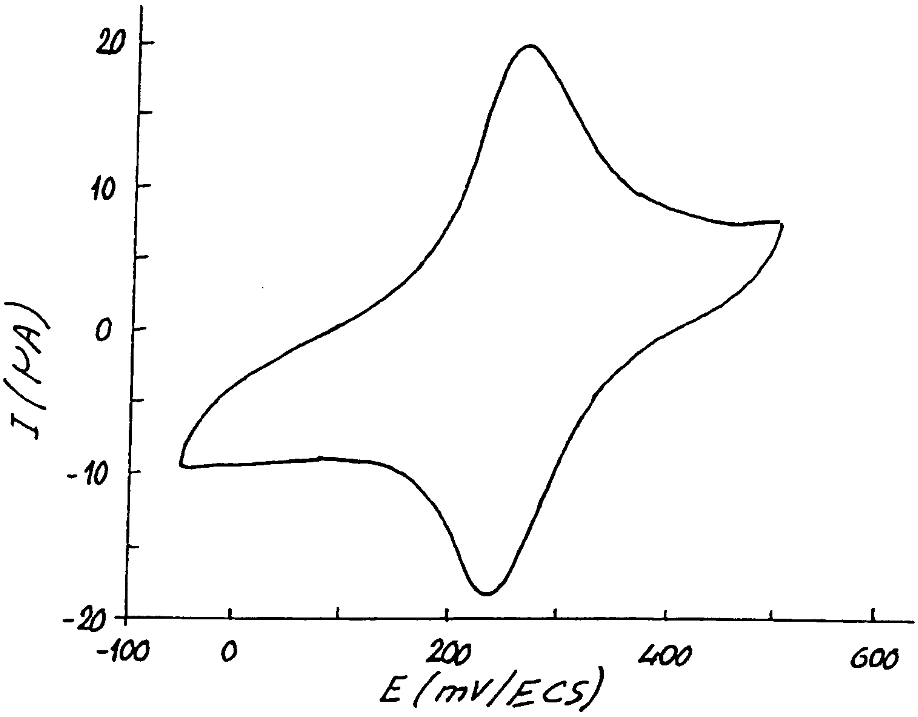
20
10
0
-10
-20
I (μA)
-100
0
200
400
600
E (mV/ECS)

FIG 7

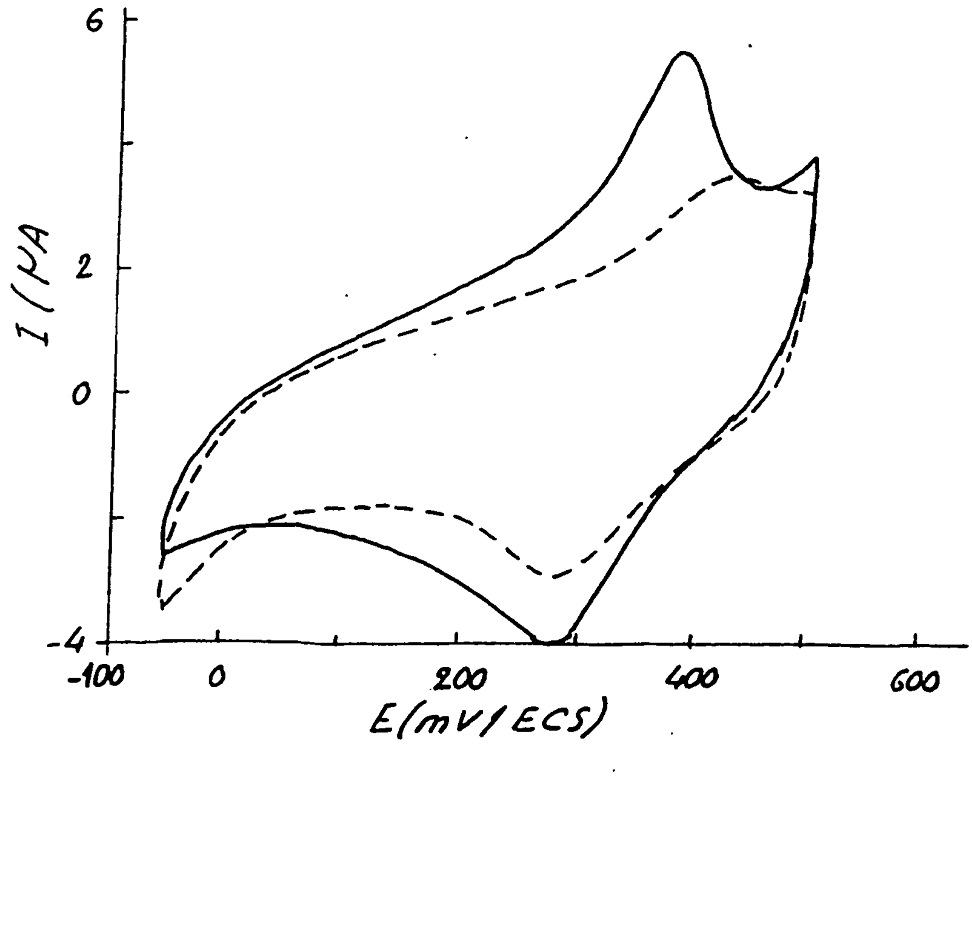
6
2
0
-4
I (μA
-100
0
200
400
600
E(mV/ECS)

FIG 9

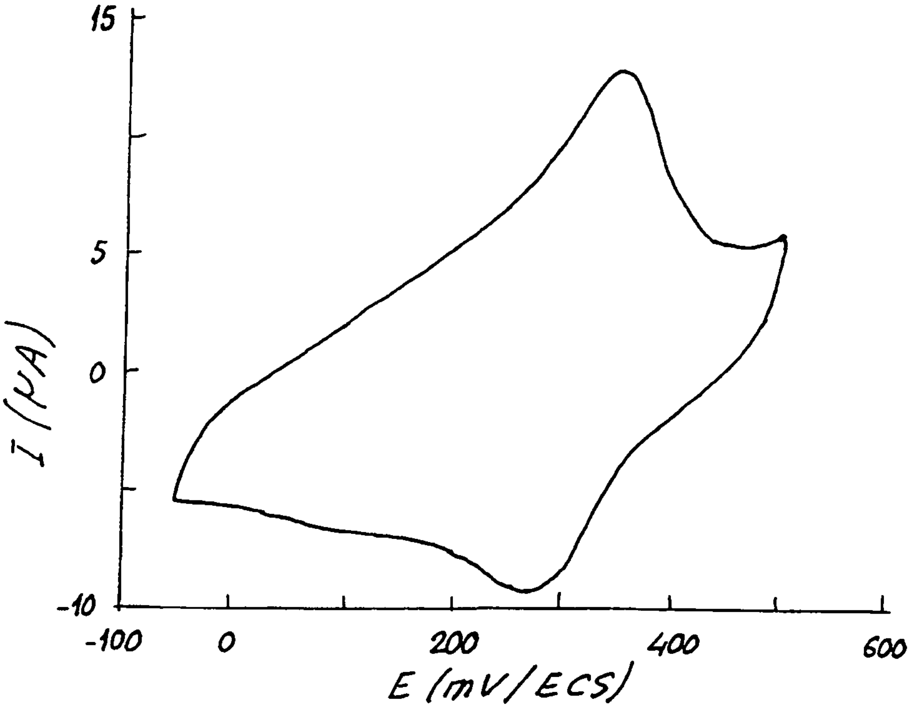
15
5
0
-10
I (μA)
-100
0
200
400
600
E (mV/ECS)

FIG 10

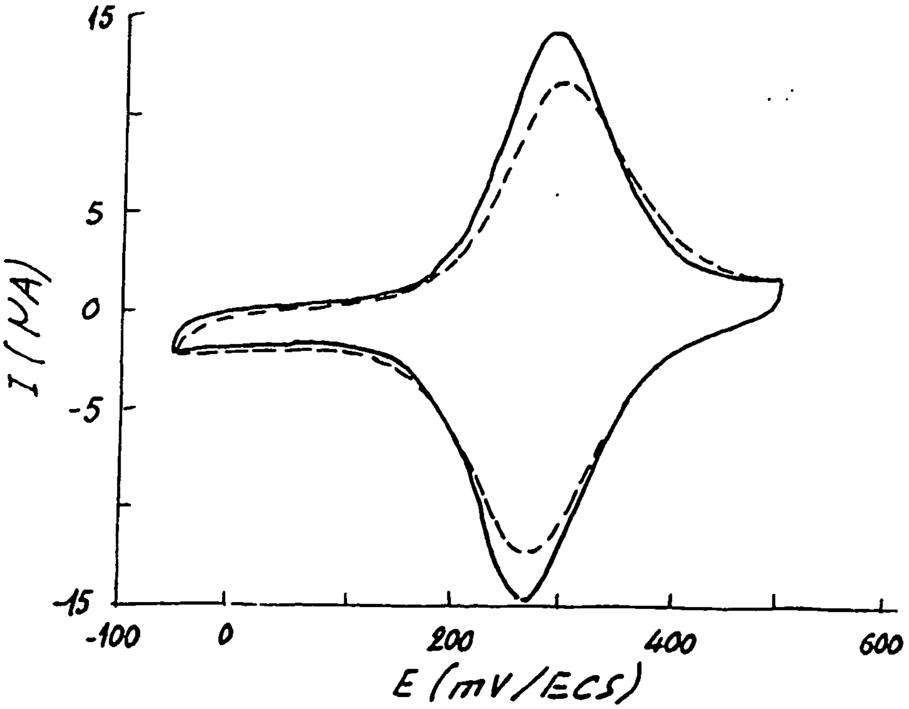
15
5
0
-5
-15
I (μA)
-100
0
200
400
600
E (mV/ECS)

FIG 11

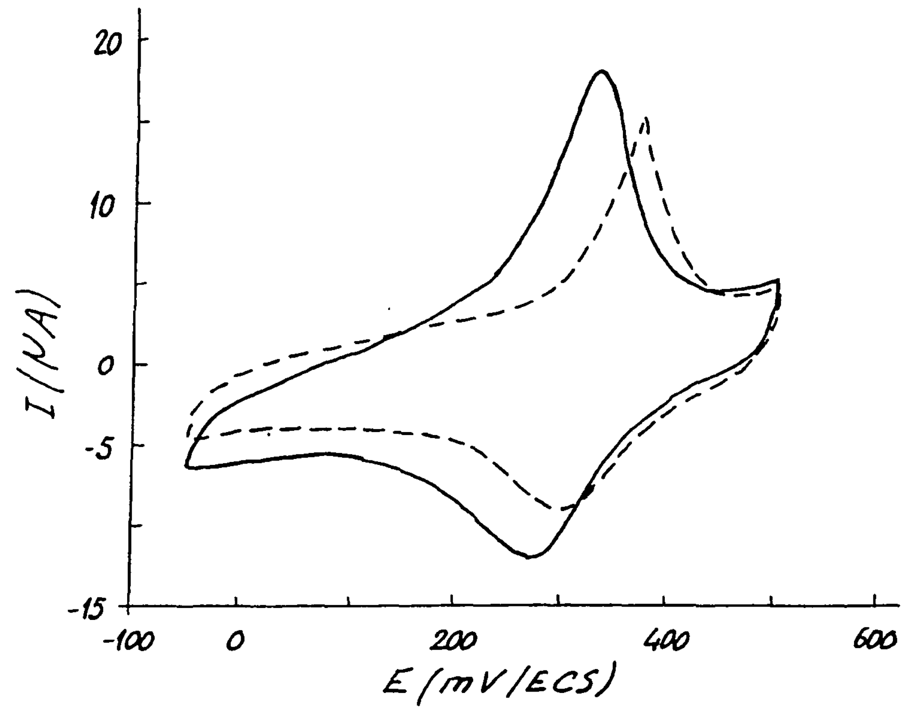
20
10
0
-5
-15
I (µA)
-100
0
200
400
600
E (mV/ECS)

FIG 12

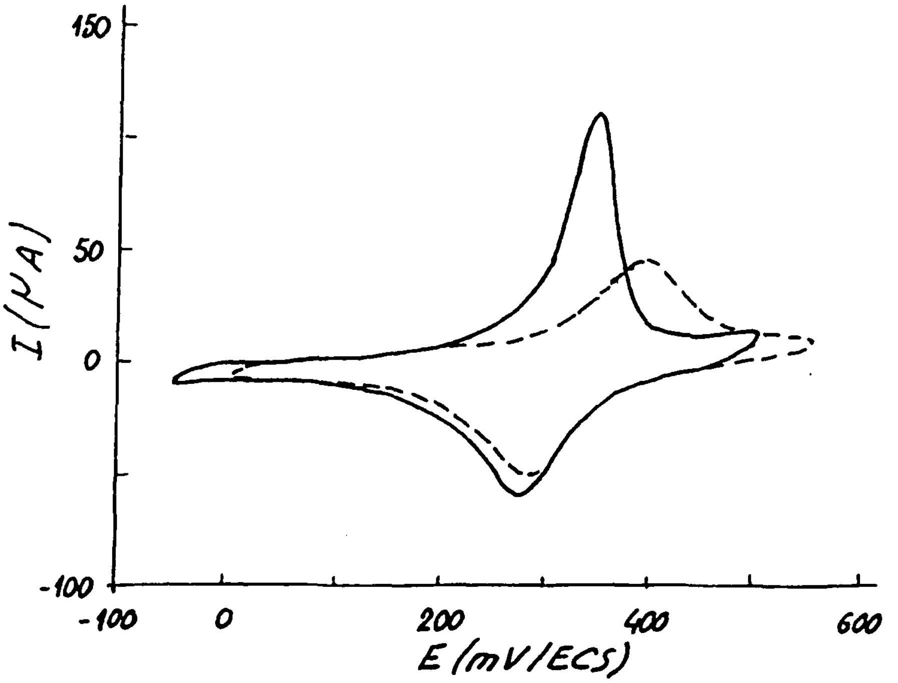
150
50
0
-100
I (µA)
-100
0
200
400
600
E (mV/ECS)

FIG 13

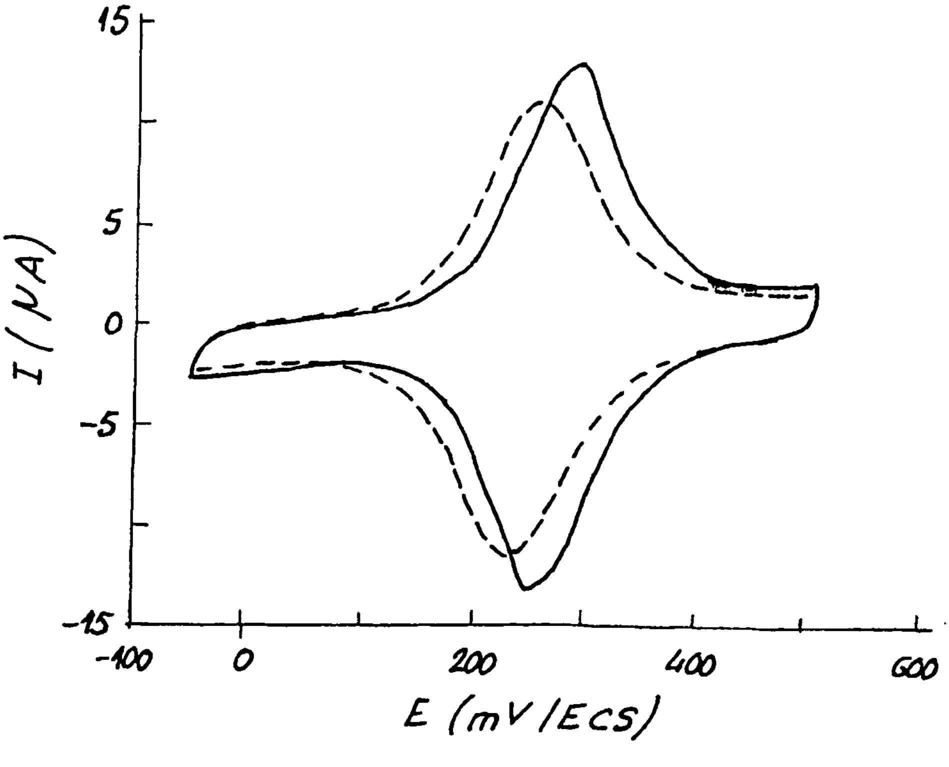

FIG 14

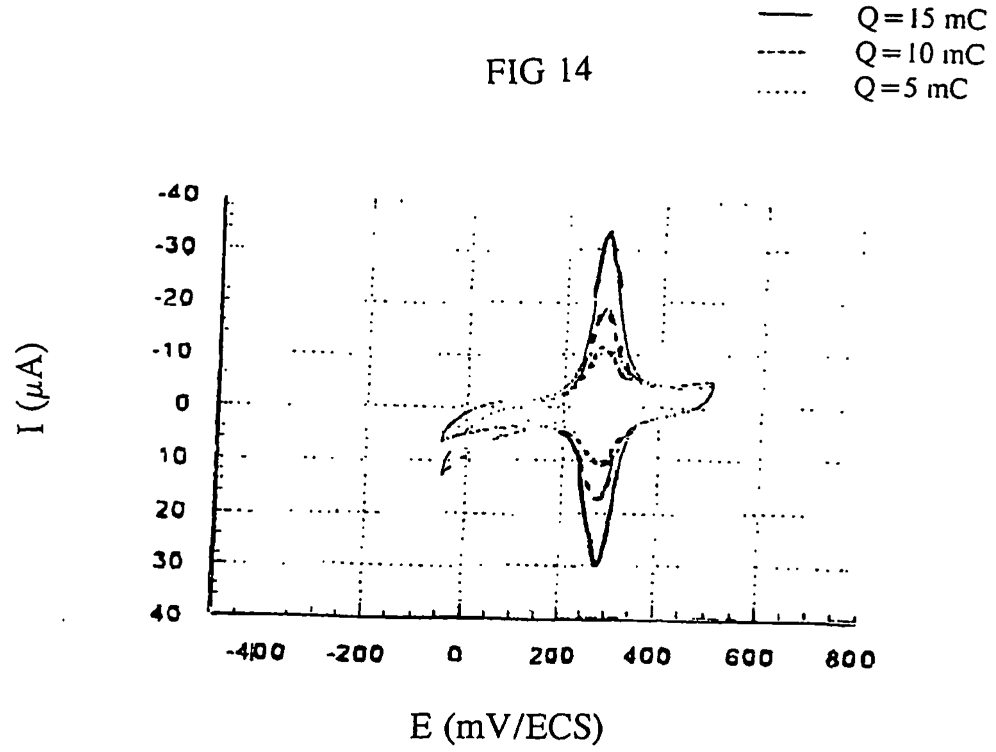
Q=15 mC
Q=10 mC
Q=5 mC
-40
-30
-20
-10
0
10
20
30
40
I (μA)
-400
-200
0
200
400
600
800
E (mV/ECS)

FIG 15

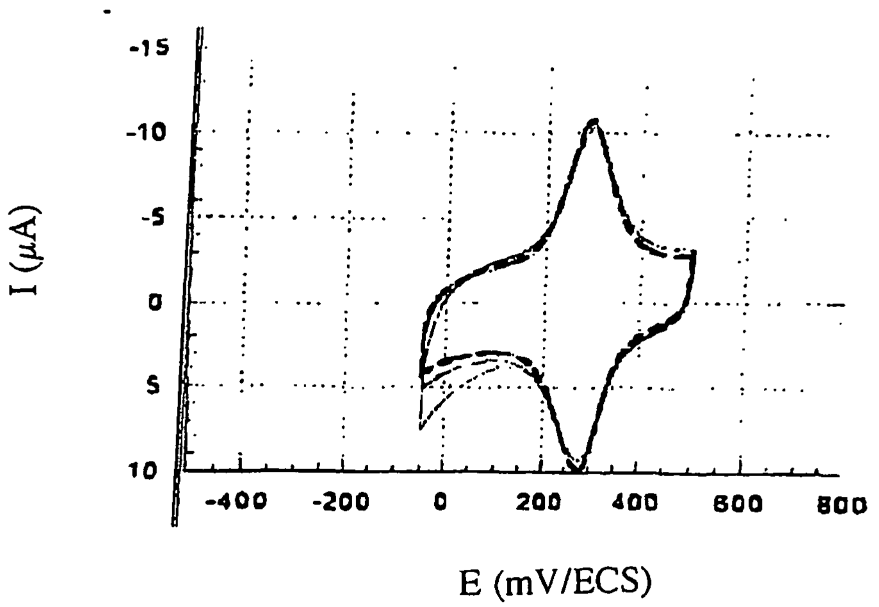
-15
-10
-5
0
5
10
I (μA)
-400
-200
0
200
400
600
800
E (mV/ECS)

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9529199 A
- WO 0031750 A1
- FR 2607507 A


### Littérature non-brevet citée dans la description


- **M. Egholm et al.** *J. Am. Chem. Soc.,* 1992, vol. 114, 1895-1897